# EUROPEAN PATENT APPLICATION

(11) **EP 4 789 626 A2**
(43) Date of publication of application: **12.08.2026**
(21) Application number: 26181158.2
(22) Date of filing: 19.08.2020
(51) Int. Cl.: A61B 17/00

(54) **IMPLANTABLE SUSPENSION DEVICE AND ASSEMBLY DEVICE**

(30) Priority: 19.08.2019 EP 19192320
(62) Document number(s) of the earlier application(s) in accordance with Art. 76 EPC: 20754776.1 / 4 017 378
(71) Applicant: ZuriMED Technologies AG, 8008 Zürich (CH)
(72) Inventor: LI, Xiang, 8008 Zürich (CH); BACHMANN, Elias, 8008 Zürich (CH); SCHUBERT, Jan, 8008 Zürich (CH)
(74) Representative: Schulz Junghans Patentanwälte PartGmbB

(57) **Abstract**

The invention relates to an implantable suspension device (1) for fixing an elongated flexible implant (G) in a desired position, comprising a suspension plate (100) and a suture element (200) engaging with the suspension plate (100), the suture element (200) comprising a first end section (201) and a second end section (202), wherein the second end section (202) comprises a connecting section (240) configured to be connected to said implant (G) or a medical textile (500).

The invention further relates to an assembly device (2) comprising a holder (400) configured to hold said suspension plate (100) and a guiding rope (300) configured to be moved together with said suture element (200) relative to the holder (400) resulting in the suture element (200) engaging with the suspension plate (100) to assemble said implantable suspension device (1) and a method for assembling an implantable suspension device (1).

## Description

The invention relates to a suspension device which is implantable into the human or animal body, particularly for connecting soft tissue (i.e. tendon or ligament) to bone, an assembly device for assembling the implantable suspension device and a method for assembling the implantable suspension device.

Implantable suspension devices, also termed cortical fixation devices (CFD), are widely used to fix soft tissue (i.e. tendon or ligament grafts) to bone during surgical procedures such as anterior cruciate ligament (ACL) reconstruction, Achilles tendon repair, biceps tendon repair, meniscus root repair or rotator cuff repair. Such devices commonly comprise a suture element used to connect the soft tissue to the device and a suspension plate or button attaching to the bone.

In the known implantable suspension devices according to the prior art, the suture element forms a closed loop, which requires the soft tissue to pass across the loop and fold in order to be connected to the suspension device, resulting in the closed loop contacting the middle part of the folded implant.

However, the other side of the folded graft is open, requiring the use of an interference screw to press-fit the implant into a bone tunnel.

Additionally, in many cases, it is not possible to fit the folded implant to the closed loop for cortical fixation.

Therefore, the objective underlying the present invention is to provide an implantable suspension device which is improved in view of the above-stated disadvantages of the prior art.

This objective is attained by the subject matter of the independent claims 1, 17, 22, 42 and 64. Embodiments of the invention are stated in sub claims 2-16, 18-21, 23-41, 43-63 and 65-71 and are described hereafter.

A first aspect of the invention relates to an implantable suspension device for fixing an elongated flexible implant (e.g. a ligament or tendon graft) or tissue (such as bone tissue) in a desired position, wherein the implantable suspension device comprises a suspension plate and a suture element engaging with the suspension plate, wherein the suture element comprises a first end section extending from the suspension plate to a first end, particularly a free first end, of the suture element, and a second end section extending from the suspension plate to a second end, particularly a free second end, of the suture element, wherein the second end section comprises a connecting section configured to be connected to the implant or to a medical textile, particularly by stitching or felting.

In particular, the implantable suspension device (also termed cortical fixation device) is suitable for fixing soft tissue, e.g. a tendon or ligament graft, to a bone. Medical applications of such suspension devices include anterior cruciate ligament (ACL) reconstruction, Achilles tendon repair, biceps tendon repair, meniscus root repair or rotator cuff repair. In particular, in case of an ACL reconstruction, the suspension plate, particularly its rear side, is configured to butt against the tibia or femur of the patient, wherein particularly the second end section extends through a bore hole drilled into the tibia or femur.

In the context of the present specification, the term suspension plate (also termed button) designates a member being configured to secure an implant, particularly a tendon or ligament graft, at its desired location, particularly in a bone tunnel. In particular, in its desired location, the suspension plate abuts a bone adjacent to (outside of) a bone tunnel harboring at least a section of the implant.

In the context of the present specification, the term suture element designates an elongated and flexible member (such as a thread, a ribbon or a wire) configured to be connected to tissue by stitching. The suture element may be from any suitable material, e.g. natural or synthetic fabric, polymers or metal.

The expression "a suture element engaging with the suspension plate" means that the suture element is mechanically connected to the suspension plate, e.g. by insertion of the suture element into at least one through-hole of the suspension plate.

In contrast to implantable suspension devices of the prior art which use a closed suture loop for connection to the middle portion of a folded implant, the suspension device according to the present invention allows to fix one end of an unfolded implant to the connecting section, such that the other end of the implant is free, particularly for connecting a further suspension device. Thus, the suspension device according to the invention can be more easily connected to an implant, and the resulting assembly is more versatile compared to the prior art.

In certain embodiments, the connecting section is positioned at the second end of the suture element.

In certain embodiments, the suture element forms a first locking loop for pressing the first end section of the suture element against the suspension plate and thereby locking the first end section with respect to the suspension plate. In particular, the first locking loop is configured to receive the first end section, such that the first end section extends through the first locking loop.

The locking loop provides a self-locking mechanism eliminating the need for separate fixing means to attach the suture element to the suspension plate.

In certain embodiments, the second end section comprises an adjustable first suspension section between the suspension plate and the connecting section, wherein the first suspension section is configured to be shortened by pulling on the first end section of the suture element, wherein particularly the first end section protrudes out of the first locking loop.

Using the first suspension section, the length of the first suspension section may be adjusted to the needs of the individual patient in an easy manner, particularly adjusted to the length of the bone tunnel.

In certain embodiments, the suspension plate comprises a front side and a rear side, which rear side faces away from the front side.

In certain embodiments, the suspension plate comprises a plurality of through-holes, each through-hole extending from the front side to the rear side of the suspension plate, wherein the suture element extends through the through-holes.

In certain embodiments, the plurality of through-holes is formed by at least three through-holes. In certain embodiments, the plurality of through-holes is formed by 3, 4 or 6 through-holes.

In certain embodiments, the suture element extends through a first-through-hole of the plurality of through-holes from the rear side to the front side of the suspension plate, with the first end ahead, particularly such that the adjustable first suspension section is formed on the rear side of the suspension plate.

In certain embodiments, the suture element further extends through a second through-hole of the plurality of through-holes from the front side to the rear side of the suspension plate, with the first end ahead, particularly such that the first locking loop is formed on the front side of the suspension plate.

In certain embodiments, the suture element further extends through a third through-hole of the plurality of through-holes from the rear side to the front side of the suspension plate with the first end ahead. In particular, the first end section of the suture element further extends through the first locking loop for clamping the first end section to the front side of the suspension plate by means of the first locking loop with a locking force.

In certain embodiments, the suture element further extends through a fourth through-hole of the plurality of through-holes from the front side to the rear side of the suspension plate with the first end ahead.

In certain embodiments, the suture element further extends through a fourth through-hole of the plurality of through-holes from the rear side to the front side of the suspension plate with the second end ahead, particularly such that the suture element forms a second suspension section between the connecting section and the suspension plate.

In certain embodiments, the suture element further extends through a fifth through-hole of the plurality of through-holes from the front side to the rear side of the suspension plate with the second end ahead, particularly such that a second locking loop for pressing the second end section against the suspension plate and thereby locking the second end section with respect to the suspension plate is formed. In particular, the second locking loop is configured to receive the second end section, such that the second end section extends through the second locking loop.

In certain embodiments, the suture element further extends through a sixth through-hole of the plurality of through-holes from the rear side to the front side of the suspension plate with the second end ahead, wherein particularly the second end section of the suture element further extends through the second locking loop for clamping the second end section of the suture element to the front side of the suspension plate by means of the second locking loop with a locking force.

In certain embodiments, the second end section comprises an adjustable second suspension section between the suspension plate and the connecting section, wherein the second suspension section is configured to be shortened by pulling on the second end section of the suture element, particularly the second end of the suture element. In particular, the second end section protrudes out of the second locking loop.

In certain embodiments, the suspension plate is connected to or integrally formed with a screw or an anchor configured to be inserted into a bone. Thereby, the suspension plate can be tightly fixed to a bone where required, while being able to easily connect soft tissue to the suspension plate, and particularly adjust the length of the first and/or second suspension section.

In certain embodiments, the suture element comprises a needle attached to the suture element, particularly to the first end or the second end. Using the needle, the connecting section may be connected to an implant without further tools.

In certain embodiments, the suture element is braided from an ultra-high molecular weight polyethylene. In certain embodiments, the suture element is co-braided from an ultra-high molecular weight polyethylene and polypropylene, polyester or polyamide.

In certain embodiments, the suspension plate, particularly the front side and/or the rear side of the suspension plate, has a surface roughness of at least 0.6 µm.

In certain embodiments, the suture element comprises or consists of a plurality of fibers (particularly braided or twisted fibers), wherein the fibers are separated from each other in the connecting section, particularly wherein each of the separated fibers comprises a fiber end or a fiber loop positioned at the second end of the suture element. Such fibers can be advantageously connected to a medical textile or an implant by felting (also termed "needle felting"), i.e., repeatedly advancing a needle comprising at least one barb through the connecting section and the medical textile or implant.

The fibers are separated from each other in the connecting section. That means that, as opposed to the remaining suture element, the fibers in the connecting section are not connected, particularly braided (i.e. entangled), twisted, knitted or felted, with neighboring fibers. Of course, the fibers may still contact neighboring fibers in the connecting section.

By means of the separated fibers of the connecting section, which are spread over a larger area than in the main section of the medical implant, pressure can be advantageously distributed over a larger area when the connecting section is connected to soft tissue thereby improving the stability of the mechanical connection. At the same time, the good tensile strength of the suspension device is retained due to the braided or twisted suture element.

In certain embodiments, the connecting section forms a fan-like structure extending in a plane parallel to an extension direction of the suture element. This advantageously increases the area of the connecting section, thereby improving the connection strength.

A second aspect of the invention relates to a medical implant comprising the implantable suspension device according to the first aspect and a medical textile, particularly comprising or consisting of a felt material, wherein the connecting section of the suture element is connected to the medical textile, and wherein the medical textile is configured to be connected to the elongated flexible implant. Such a medical textile advantageously improves the connection of the suture element to the implant.

In certain embodiments, the connecting section is connected to the medical textile by stitches.

In certain embodiments, the suture element comprises or consists of a plurality of fibers, wherein the fibers are separated from each other in the connecting section, and wherein the separated fibers of the connecting section extend into and/or through the medical textile to connect the suture element of the implantable suspension device to the medical textile. This type of connection can be particularly achieved by advancing a needle having at least one barb repeatedly through the connecting section and the medical textile, resulting in a very strong connection using a fast and simple procedure.

In certain embodiments, the medical textile extends along a plane.

In certain embodiments, the medical textile comprises a flat shape.

In certain embodiments, the connecting section of the suture element forms a fan-like structure extending in a plane parallel to an extension direction of the suture element, wherein the suture element is connected to the medical textile, such that the first medical implant extends from the medical textile parallel to the plane of the medical textile. This further increases the area of the connecting section, contributing to the connection strength.

In certain embodiments, the medical textile comprises a tubular shape, wherein the medical textile comprises an inner surface configured to be connected to the elongated flexible implant. This allows an especially tight connection to implants having an elongated shape, such as tendons.

A third aspect of the invention relates to an assembly device for assembling the implantable suspension device according to the first aspect of the invention, wherein the assembly device comprises a holder configured to hold the suspension plate of the implantable suspension device and a guiding rope (or guiding wire) configured to be moved together with the suture element of the implantable suspension device relative to the holder, resulting in the suture element engaging with the suspension plate to assemble the implantable suspension device.

Therein, the term guiding rope refers to an elongated flexible member from any suitable material configured to guide the suture element for engagement with the suspension plate.

In certain embodiments, the guiding rope extends through at least one through-hole of the plurality of through-holes of the suspension plate held by the holder, such that the guiding rope can be moved through the at least one through-hole together with the suture element, resulting in the suture element engaging with the suspension plate.

In certain embodiments, the assembly device comprises a first pin and a second pin, wherein the holder is configured to hold the suspension plate between the first pin and the second pin, and wherein the assembly device is configured such that the guiding rope can be moved (or slid) around the first pin and the second pin together with the suture element resulting in the suture element engaging with the suspension plate.

The holder is configured to hold the suspension plate between the first pin and the second pin. In other words, the holder is configured to hold the suspension plate in a position intersecting an imaginary line between the first pin and the second pin. Therein, the suspension plate is particularly spaced apart from the first pin and the second pin.

In the context of the present specification, the expression "move around a pin" means that the guiding rope or suture element is moved while it is in contact with at least a part of the circumference of the pin, wherein particularly the guiding rope or suture element extends along an angled path around the pin. In other words, the guiding rope or suture element does not have to be wound completely around the circumference (360°) to be "moved around the pin".

The guiding rope is particularly in contact with the first pin and the second pin, while it is moved around the first and the second pin.

The first pin and the second pin ensure that the guiding rope and the suture element slide through the through-holes of the suspension plate without entangling.

In certain embodiments, the holder is configured to hold the suspension plate between the first pin and the second pin such that the first pin faces the front side of the suspension plate and the second pin faces the rear side of the suspension plate.

In certain embodiments, the guiding rope extends successively through the first through-hole from the rear side to the front side of the suspension plate, around the first pin, through the second through-hole from the front side to the rear side of the suspension plate, around the second pin and through the third through-hole from the rear side to the front side of the suspension plate.

In certain embodiments, the guiding rope extends successively through the fourth through-hole from the rear side to the front side of the suspension plate, around the first pin, through the fifth through-hole from the front side to the rear side of the suspension plate, around the second pin and through the sixth through-hole from the rear side to the front side of the suspension plate.

In certain embodiments, the guiding rope forms a loop configured to enlace the suture element, such that the loop can be moved together with the suture element relative to the holder resulting in the suture element engaging with the suspension plate.

Therein, the loop enlacing the suture element means that the suture element extends through the loop.

In certain embodiments, the guiding rope comprises an inner volume extending between a first end and a second end of the guiding rope, wherein the guiding rope is configured to receive the suture element in the inner volume, such that the guiding rope can be moved together with the suture element disposed in the inner volume relative to the holder resulting in the suture element engaging with the suspension plate.

In certain embodiments, the guiding rope forms a funnel at the first end, wherein the funnel defines an opening for receiving the suture element in the inner volume.

In certain embodiments, the funnel is characterized by an expanded state and a collapsed state, wherein the opening has a first diameter in the expanded state and a second diameter in the collapsed state, wherein the first diameter is larger than the second diameter.

In certain embodiments, the assembly device comprises a holding part comprising the holder and a casing part comprising a wall configured to encase the holder when the holding part and the casing part are assembled.

In certain embodiments, the wall comprises an opening for inserting and/or extracting the suture element by means of the guiding rope, particularly the first end and/or the second end of the suture element.

In certain embodiments, the holding part comprises the first pin and the second pin.

In certain embodiments, the casing part comprises a first hole configured to receive the first pin of the holding part and a second hole configured to receive the second pin of the holding part to assemble the holding part and the casing part.

In certain embodiments, the assembly device comprises a tool for implanting the implantable suspension device at a desired location in a human or animal body. For example, such a tool may be used to place the suspension plate at the desired location adjacent to a bone tunnel and adjust the length of the first and/or second suspension section to position the implant in the bone tunnel.

In certain embodiments, the tool comprises a guide bar extending along a first axis configured to guide the guiding rope and/or the suture element, wherein the guide bar comprises a slot extending along the first axis for receiving the guiding rope and/or the suture element, particularly the first end or the second end of the suture element.

In certain embodiments, the guide bar comprises a recess or a hole for receiving the first pin or the second pin.

In certain embodiments, the guide bar comprises a tip comprising a notch for receiving the suspension plate.

In certain embodiments, the tool comprises a handle for holding the tool, wherein the handle is connected to the guide bar.

In certain embodiments, the handle comprises a groove for inserting the guiding rope and/or the suture element when at least a part of the guiding rope and/or the suture element is arranged in the slot.

In certain embodiments, the tool is separable into a handle part comprising the handle and a tip part comprising at least a part of the guiding bar.

The separable tip part may be preassembled with the positioning part holding the suspension plate and the guiding rope in place and provided as a disposable unit (which is particularly presterilized and packaged in a sterile environment), while the handle part can be a re-usable unit.

In certain embodiments, the handle part and the tip part comprise an inner thread and an outer thread corresponding to the inner thread. In certain embodiments, the handle part comprises an inner thread and the tip part comprises an outer thread corresponding to the inner thread. In certain embodiments, the tip part comprises an inner thread and the handle part comprises an outer thread corresponding to the inner thread. An inner thread corresponding to an outer thread means that the part comprising the outer thread can be connected to the part comprising the inner thread by screwing the outer thread into the inner thread.

In certain embodiments, the assembly device is configured to clamp the suture element in the slot.

In certain embodiments, the assembly device comprises an actuating element that can be actuated to clamp the suture element in the slot.

In certain embodiments, the assembly device comprises a self-locking clamp for clamping the suture element when the suture element is received in the slot, wherein particularly the assembly device comprises an actuating element configured to unclamp the suture element.

In certain embodiments, the assembly device comprises a first part comprising a fixing element for attaching the guiding rope, particularly a first end of the guiding rope, to the first part, and a second part comprising the holder, wherein the second part is rotatable about a rotation axis relative to the first part, such that the guiding rope can be moved together with the suture element relative to the holder when the second part is rotated relative to the first part, resulting in the suture element engaging with the suspension plate.

In certain embodiments, the first part further comprises a seventh pin for guiding the guiding rope towards the fixing element.

By rotating the second part relative to the first part, the guiding rope can be moved in a spacesaving manner to achieve engagement of the suture element with the suspension plate.

In certain embodiments, the first pin and the second pin are comprised in, particularly arranged on, the second part.

In certain embodiments, the assembly device further comprises a third pin and a fourth pin comprised in, particularly arranged on, the second part.

In certain embodiments, the third pin is arranged between the first pin and the second pin along a periphery around the holder.

In certain embodiments, the fourth pin is arranged between the first pin and the second pin and opposite the third pin along the periphery.

In certain embodiments, the guiding rope is arrangeable along the periphery around the first pin, the second pin, the third pin and/or the fourth pin when the second part is rotated relative to the first part.

In certain embodiments, the assembly device further comprises a fifth pin for guiding the guiding rope and/or the suture element from the holder towards the fixing element.

In certain embodiments, the fifth pin is arranged on the second part adjacent to the first pin radially outward from the first pin with respect to the rotation axis.

In certain embodiments, the assembly device further comprises a sixth pin, particularly arranged on the second part.

In certain embodiments, the fifth pin and the sixth pin are arranged such that the guiding rope and/or the suture element can be guided from the holder towards the fixing element between the fifth pin and the sixth pin when the second part is rotated relative to the first part.

In certain embodiments, the assembly device comprises a lid part comprising a first handle, wherein the lid part is configured to be coupled to the second part, wherein the first part comprises a second handle, such that the second part can be rotated relative to the first part by moving the first handle relative to the second handle.

In certain embodiments, the lid part comprises at least a first hole and a second hole, wherein the first hole and the second hole are each configured to receive one of the first pin, the second pin, the third pin, the fourth pin, the fifth pin or the sixth pin to couple the lid part to the second part. In certain embodiments, the lid part comprises a first hole, wherein the first hole is configured to receive the first pin, the second pin, the third pin, the fourth pin, the fifth pin or the sixth pin to couple the lid part to the second part. In certain embodiments, the lid part comprises a second hole, wherein the second hole is configured to receive the first pin, the second pin, the third pin, the fourth pin, the fifth pin or the sixth pin to couple the lid part to the second part.

A fourth aspect of the invention relates to a method for assembling an implantable suspension device, particularly according to the first aspect of the invention, wherein a suspension plate and a suture element are provided, wherein the suture element is engaged with or connected to the suspension plate, such that a first end section of the suture element extends from the suspension plate to a first end of the suture element and a second end section of the suture element extends from the suspension plate to a second end of the suture element.

In certain embodiments, a connecting section comprised in the second end section is connected, particularly stitched, to an elongated flexible implant or tissue. In particular, connecting the connecting section to the implant or tissue is performed ex vivo. Connection to the implant may be performed prior to or after assembling the suture element with the suspension plate.

In certain embodiments, a first locking loop is formed from the suture element, wherein the first locking loop presses the first end section of the suture element against the suspension plate, thereby locking the first end section with respect to the suspension plate.

In certain embodiments, an adjustable first suspension section of the suture element between the suspension plate and the connecting section is shortened by pulling on the first end section of the suture element, wherein particularly the first end section protrudes out of the first locking loop.

In certain embodiments, the suture element is inserted into a plurality of through-holes of the suspension plate, wherein the through-holes extend from a front side of the suspension plate to a rear side of the suspension plate facing away from the front side. In certain embodiments, the suture element is inserted into at least three through-holes, wherein the through-holes extend from a front side of the suspension plate to a rear side of the suspension plate facing away from the front side. In certain embodiments, the suture element is inserted into 3, 4 or 6 through-holes, wherein the through-holes extend from a front side of the suspension plate to a rear side of the suspension plate facing away from the front side.

In certain embodiments, the suture element is inserted into a first-through-hole of the plurality of through-holes from the rear side to the front side of the suspension plate with the first end ahead, particularly such that the adjustable first suspension section is formed on the rear side of the suspension plate.

In certain embodiments, the suture element is further inserted into a second through-hole of the plurality of through-holes from the front side to the rear side of the suspension plate with the first end ahead, particularly such that the first locking loop is formed on the front side of the suspension plate.

In certain embodiments, the suture element is further inserted into a third through-hole of the plurality of through-holes from the rear side to the front side of the suspension plate with the first end ahead. In particular, the first end section of the suture element is further inserted into the first locking loop for clamping the first end section to the front side of the suspension plate by means of the first locking loop with a locking force.

In certain embodiments, the suture element is further inserted into a fourth through-hole of the plurality of through-holes from the front side to the rear side of the suspension plate with the first end ahead.

In certain embodiments, the suture element is further inserted into a fourth through-hole of the plurality of through-holes from the rear side to the front side of the suspension plate with the second end ahead, particularly such that the suture element forms a second suspension section between the connecting section and the suspension plate.

In certain embodiments, the suture element is further inserted into a fifth through-hole of the plurality of through-holes from the front side to the rear side of the suspension plate with the second end ahead, particularly such that a second locking loop for pressing the second end section against the suspension plate and thereby locking the second end section with respect to the suspension plate is formed.

In certain embodiments, the suture element is further inserted into a sixth through-hole of the plurality of through-holes from the rear side to the front side of the suspension plate with the second end ahead. In particular, the second end section of the suture element is further inserted into the second locking loop for clamping the second end section of the suture element to the front side of the suspension plate by means of the second locking loop with a locking force.

In certain embodiments, an adjustable second suspension section of the suture element between the connecting section and the suspension plate is shortened by pulling on the second end section, particularly the second end, of the suture element, wherein particularly the second end section protrudes out of the second locking loop.

In certain embodiments, the connecting section is connected to the elongated flexible implant or tissue by means of a needle attached to the suture element, particularly to the first end or the second end.

In certain embodiments, a guiding rope is moved together with the suture element resulting in the suture element engaging with the suspension plate to assemble the implantable suspension device.

In certain embodiments, the guiding rope is inserted into at least one through-hole of the plurality of through-holes of the suspension plate, wherein the guiding rope is subsequently moved through the at least one through-hole together with the suture element, resulting in the suture element engaging with the suspension plate.

In certain embodiments, the guiding rope is moved around a first pin and a second pin together with the suture element during engagement of the suture element with the suspension plate, wherein particularly the suspension plate is arranged between the first pin and the second pin, such that the first pin faces the front side of the suspension plate and the second pin faces the rear side of the suspension plate.

In certain embodiments, the guiding rope is successively inserted into the first through-hole from the rear side to the front side of the suspension plate, around the first pin, into the second through-hole from the front side to the rear side of the suspension plate, around the second pin and into the third through-hole from the rear side to the front side of the suspension plate.

In certain embodiments, the guiding rope is successively inserted into the fourth through-hole from the rear side to the front side of the suspension plate, around the first pin, into the fifth through-hole from the front side to the rear side of the suspension plate, around the second pin and into the sixth through-hole from the rear side to the front side of the suspension plate.

In certain embodiments, the guiding rope forms a loop enlacing the suture element, wherein the loop is moved together with the suture element resulting in the suture element engaging with the suspension plate.

In certain embodiments, the suture element is received in an inner volume of the guiding rope extending between a first end and a second end of the guiding rope, wherein the guiding rope is moved together with the suture element disposed in the inner volume resulting in the suture element engaging with the suspension plate.

In certain embodiments, the suture element is received in the inner volume through an opening of a funnel formed by the guiding rope at the first end of the guiding rope.

In certain embodiments, the funnel is collapsed from an expanded state to a collapsed state during engagement of the suture element with the suspension plate, wherein the opening has a first diameter in the expanded state and a second diameter in the collapsed state, wherein the first diameter is larger than the second diameter.

In certain embodiments, after assembly of the implantable suspension device, the implantable suspension device is mounted, particularly automatically, on a tool for implanting the implantable suspension device into a human or animal body.

In certain embodiments, the guiding rope, particularly a first end of the guiding rope, is attached to a fixing element, wherein the fixing element is moved along a periphery around the suspension plate, such that the guiding rope together with the suture element is moved relative to the suspension plate resulting in the suture element engaging with the suspension plate.

In certain embodiments, the guiding rope is moved along the periphery around the first pin and the second pin.

In certain embodiments, the guiding rope is moved along the periphery around the first pin, the second pin, a third pin and a fourth pin, the third pin being arranged between the first pin and the second pin along the periphery and the fourth pin being arranged between the first pin and the second pin and opposite the third pin along the periphery.

In certain embodiments, the guiding rope is further moved around a fifth pin, wherein the fifth pin guides the guiding rope and/or the suture element towards the fixing element, wherein the fifth pin is arranged adjacent to the first pin.

In certain embodiments, the guiding rope is further moved around a sixth pin, wherein the guiding rope and/or the suture element is guided towards the fixing element between the fifth pin and the sixth pin.

A fifth aspect of the invention relates to a method for assembling a medical implant, particularly according to the second aspect of the invention, comprising the steps of providing a suture element comprising a connecting section, providing a medical textile, particularly comprising or consisting of a felt material, connecting the connecting section of the suture element to the medical textile, providing a suspension plate, and engaging or assembling the suture element with the suspension plate, particularly by the method according to the fourth aspect of the invention.

In certain embodiments, the connecting section of the suture element is connected to the medical textile by stitching.

In certain embodiments, the suture element comprises or consists of a plurality of fibers, wherein the fibers are separated from each other in the connecting section, and wherein the separated fibers of the connecting section are advanced into and/or through the medical textile to connect the suture element of the implantable suspension device to the medical textile.

In certain embodiments, the suture element is connected to the medical textile by repeatedly advancing a needle comprising at least one barb through the connecting section and the medical textile to advance the separated fibers of the connecting section into and/or through the medical textile.

In certain embodiments, the medical textile and/or the suture element is connected to an elongated flexible implant, particularly outside of a human or animal body.

In certain embodiments, the medical textile and/or the suture element is connected to the elongated flexible implant, particularly outside of the human or animal body, after connecting the connecting section of the suture element to the medical textile.

In certain embodiments, the medical textile and/or the suture element is connected to the elongated flexible implant, particularly outside of the human or animal body, prior to engaging or assembling the suture element with the suspension plate.

In certain embodiments, the suture element is stitched to the implant, particularly outside of the human or animal body.

In certain embodiments, the medical textile is connected to the implant by repeatedly advancing a needle comprising at least one barb through the medical textile and the elongated flexible implant, particularly outside of the human or animal body.

In certain embodiments, the suture element is connected to the implant by repeatedly advancing a needle comprising at least one barb through the connecting section of the suture element and the elongated flexible implant, particularly outside of the human or animal body.

In certain embodiments, the medical textile is at least partially wrapped around the implant prior to connecting the medical textile to the elongated flexible implant, particularly wherein the medical textile is wrapped around the elongated flexible implant, such that the medical textile assumes a tubular shape, wherein the elongated flexible implant is connected to an inner surface of the medical textile.

Further features and advantages of the invention shall be described by means of detailed descriptions of embodiments with reference to the Figures, wherein
- Fig. 1: shows the assembly of an implantable suspension device according to a first embodiment of the invention;
- Fig. 2: shows the assembly of an implantable suspension device according to a second embodiment of the invention;
- Fig. 3: shows the assembly of an implantable suspension device according to a third embodiment of the invention;
- Fig. 4: shows a further embodiment of the implantable suspension device according to the invention, where the suspension plate is integrally formed with a bone screw;
- Fig. 5: shows a further embodiment of the implantable suspension device according to the invention, where the suspension plate is integrally formed with a bone anchor;
- Fig. 6: schematically shows the threading of the suture element through the through-holes of the suspension plate by means of a looped guiding rope according to the invention;
- Fig. 7: shows a hollow guiding rope with a funnel structure for engaging the suture element with the suspension plate of the suspension device according to the invention;
- Fig. 8: shows the assembly of the suture element with the suspension plate using an assembly device according to the invention;
- Fig. 9: shows an assembly device according to the invention including a tool for implanting the suspension device comprising a handle and a guide bar;
- Fig. 10: shows details of the assembly device according to Fig. 9;
- Fig. 11: shows the disassembly of the assembly device according to Fig. 9 after engagement of the suture element with the suspension plate;
- Fig. 12: shows an assembly device according to the invention which is separable into a handle part and a tip part;
- Fig. 13: shows details of the tip part according to Fig. 12;
- Fig. 14: shows a further embodiment of an assembly device according to the invention comprising a first part and a second part which can be rotated with respect to each other;
- Fig. 15: shows details of the assembly device according to Fig. 14;
- Fig. 16: shows the assembly of a medical implant according to an embodiment of the invention;
- Fig. 17: shows the assembly of a medical implant according to a further embodiment of the invention.

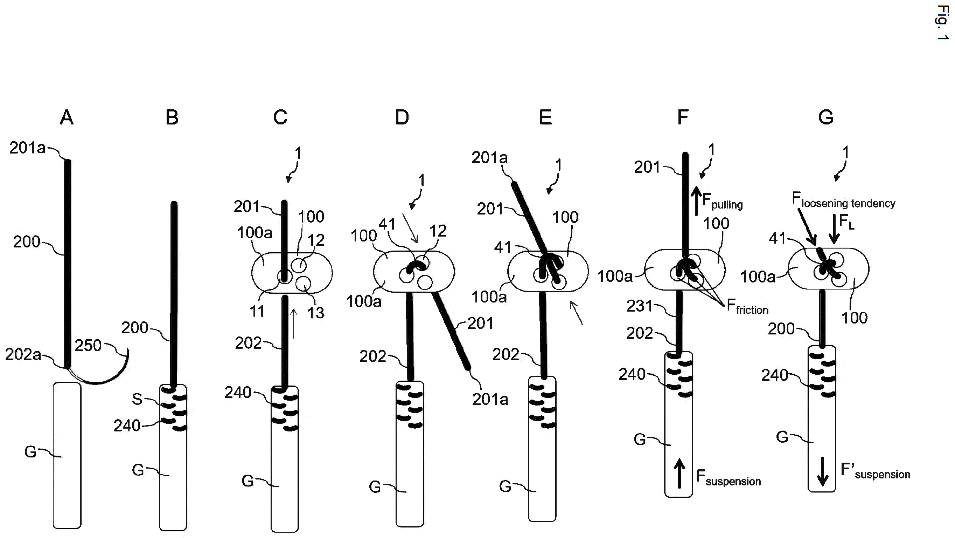

Fig. 1A-G show assembly steps of a first embodiment of the implantable suspension device 1 according to the invention. The implantable suspension device 1 comprises a suspension plate 100 and a suture element 200.

As shown in Fig. 1 A, the suture element 200 comprises a first free end 201a and a second free end 202a. A connecting section 240 disposed at the second end 202a of the suture element 200 is connected to an implant G, particularly a soft tissue such as a tendon or ligament, by stitches S (Fig. 1B). For example, the connecting section 240 of the suture element 200 may be connected to the implant G by means of a needle 250 attached to the second end 202a of the suture element 200 as shown in Fig. 1A. After stitching the connecting section 240 to the implant G, the needle 250 may be removed by cutting the suture element 200.

The first end 201a is then advanced through a first through-hole 11 of the suspension plate 100 from a rear side 100b (not shown in Fig. 1) to a front side 100a (Fig. 1C). In this manner, a first end section 201 protruding from the first through-hole 11 on the front side 100a of the suspension plate 100 and a second end section 202 disposed between the implant G and the rear side 100b of the suspension plate 100 are formed.

Subsequently, the first end 201a of the suture element 200 is inserted into a second through hole 12 of the suspension plate 100 from the front side 108 to the rear side 100 B (Fig. 1D), such that a first locking loop 41 is formed on the front side 100a of the suspension plate 100 between the first through-hole 11 and the second through-hole 12.

In the next step which is shown in Fig. 1E, the first end 201a of the suture element 200 is inserted into a third through-hole 13 from the rear side 200b to the front side 100a of the suspension plate 100 and advanced through the first locking loop 41.

The final assembly with a tightened first locking loop 41 pressing the first end section 201 against the front side 100a of the suspension plate 100 is shown in Fig. 1F.

Furthermore, a first suspension section 231 is formed between the rear side 100b of the suspension plate 100 and the connecting section 240 stitched to the implant G (Fig. 1F). The length of the first suspension section 231 may be adjusted by pulling on the first end section 201 of the suture element 200 protruding from the first locking loop 41 on the front side 100a of the suspension plate 100 with a pulling force F_{pulling} (Fig. 1F). During the pulling process, the pulling force F_{pulling} equals the sum of the suspension force Fₛᵤₛₚₑₙₛᵢₒₙ acting on the implant G via the first suspension section 231 and the friction force F_{friction} **(F_{pulling}**= **Fₛᵤₛₚₑₙₛᵢₒₙ** + F_{friction}).

As illustrated in Fig. 1 G, when the first end section 201 is released, the first end section 201 is locked by the first locking loop 41 from loosening. In this situation, the suspension force F'ₛᵤₛₚₑₙₛᵢₒₙ equals the sum of the loosening tendency force F_{loosening tenancy} and **the** friction force F'friction **(F'ₛᵤₛₚₑₙₛᵢₒₙ** = F_{loosening **tendency**} + F'friction). The locking force F_{locking} equals the friction coefficient µ_{friction} times the suspension force F'suspension. **(F_{locking}** = **µ_{friction} x F'suspension).** Because the locking force F_{locking} **is** higher than the loosening tendency force F_{loosening tendency}, **(F_{locking} > F_{loosening tendency}),** the self-locking suspension mechanism is achieved, tightly fixing the suture element 200 to the suspension plate 100.

In particular, in order to achieve a friction coefficient which is sufficient to provide a self-locking mechanism, the suture element 200 may be braided from an ultra-high molecular weight polyethylene or co-braided from an ultra-high molecular weight polyethylene and polypropylene, polyester or polyamide, and the front side 100a and/or the rear side 100b of the suspension plate may have a surface roughness of at least 0.6 µm. For example, the suture element 200 may have a diameter of about 0.5 to 0.7 mm, and the first through-hole 11, the second through-hole 12 and/or the third through-hole may have a diameter of about 1.1 mm.

After adjusting the length of the first suspension section 231, the first end section 201 may be shortened, e.g., by cutting, resulting in the configuration shown in Fig. 1G.

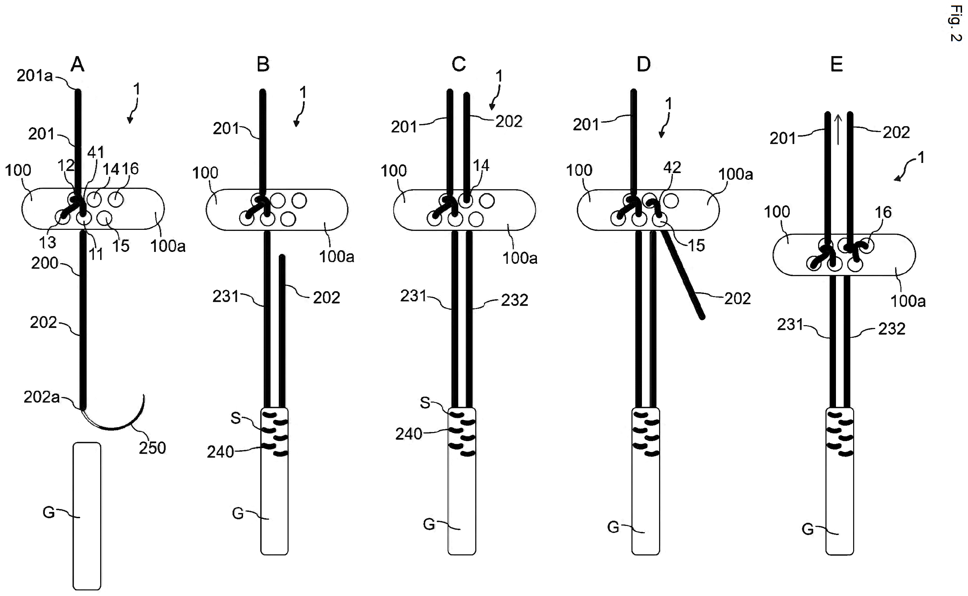

Fig. 2A-E shows the assembly of a further embodiment of an implantable suspension device 1 according to the invention comprising a suspension plate 100 with six through-holes 11, 12, 13, 14, 15, 16 and a suture element 200 with a first suspension section 231 and a second suspension section 232. As shown in Fig. 2A, the suture element 200 is particularly engaged with the suspension plate 100 prior to connecting the suture element 200 to the implant G. This pre-assembly is performed as depicted in Fig. 1, wherein the suture element 200 is advanced with the first free end 201a ahead through the first through-hole 11 from the rear side 100b to the front side 100a, through the second through-hole 12 from the front side 100a to the rear side 100b, thereby forming the first locking loop 41, and through the third through-hole 13 from the rear side 100b to the front side 100a and through the first locking loop 41, such that the first end section 201 protrudes from the first locking loop 41 at the front side 100a of the suspension plate 100. Next, a connecting section 240 of the second end section 202 protruding from the rear side 100 B of the suspension plate 100 is connected to the implant G by stitches S. In contrast to the embodiment shown in Fig. 1, the connecting section 240 is not positioned at the second end 202a of the suture element 200. Accordingly, a part of the second end section 202 extends between the connecting section 240 and the second end 202a (Fig. 2B). However, in the same manner as in the embodiment of Fig. 1, the connecting section 240 of the suture element 200 may be stitched to the implant G by means of a needle 250 attached to the second end 202a of the suture element 200 as shown in Fig. 2A. After stitching the connecting section 240 to the implant G, the needle 250 may then be removed by cutting the suture element 200.

To form the assembly shown in Fig. 2E, the second end section 202 is inserted into the fourth through-hole 14 from the rear side 100b to the front side 100a of the suspension plate 100, with the second end 202a ahead (Fig. 2C).

Subsequently, the second end section 202 is advanced through a fifth through-hole 15 from the front side 100a to the rear side 100b of the suspension plate 100 with the second end 202a ahead, thereby forming a second locking loop 42 on the front side 100a of the suspension plate 100 (Fig. 2D).

The second end section 202 is then inserted into a sixth through-hole 16 from the rear side 100b to the front side 100a of the suspension plate 100 with the second end 202a ahead and advanced through the second locking loop 42 on the front side 100a of the suspension plate 100 (Fig. 2E).

Thereby, a first suspension section 231 of the suture element 200 protruding from the rear side 100b of the suspension plate 200 is formed between the first through-hole 11 and the implant G, and a second suspension section 232 of the suture element 200 protruding from the rear side 100b of the suspension plate 200 is formed between the implant G and the fourth through-hole 14.

In the final assembly depicted in Fig. 2E, the first end section 201 of the suture element 200 protrudes from the first locking loop 41 on the front side 100a of the suspension plate 100, and the second end section 202 protrudes from the second locking loop 42 on the front side 100a of the suspension plate 100.

The length of the first and second suspension strand 231, 232 can then be adjusted independently of each other by pulling on the first end section 201 or the second end section 202, respectively (see arrow in Fig. 2E). Of course, the length of both suspension strands 231, 232 can also be adjusted simultaneously.

The self-locking mechanism of the first locking loop 41 and the second locking loop 42 functions in the same way as described above for the embodiment shown in Fig. 1 and the same forces act on each suspension strand 231, 232.

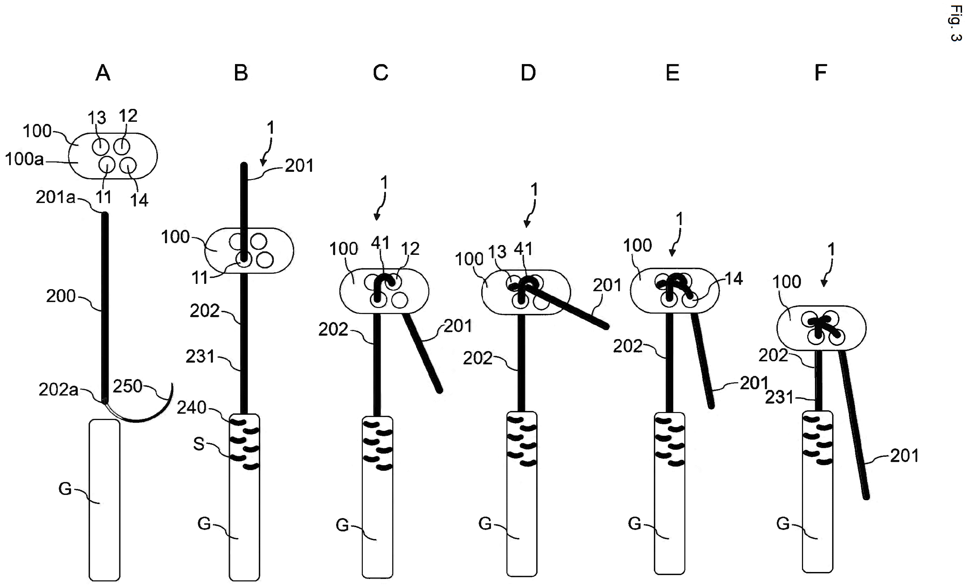

Fig. 3A-F shows the assembly of a further embodiment of the implantable suspension device 1 according to the present invention, wherein the suspension plate 100 has four through-holes 11, 12, 13, 14, and the first end section 201 protrudes from the suspension plate 100 on the same side as the first suspension strand 231.

As depicted in Fig. 3A and 3B, the suture element 200 is attached by stitches S to the implant G using a connecting section 240 positioned on the second free end 202a of the suture element 200, similar to the embodiment of Fig. 1. As described above, the connecting section 240 of the suture element 200 may be stitched to the implant G by means of a needle 250 attached to the second end 202a of the suture element 200 as shown in Fig. 3A. After stitching the connecting section 240 to the implant G, the needle 250 may then be removed by cutting the suture element 200. Of course, this step may also be performed after assembling the suspension plate 100 with the suture element 200.

The first free end 201a of the suture element 200 is inserted into the first through-hole 11 of the suspension plate 100 from the rear side 100b to the front side 100a (Fig. 3B), into the second through-hole 12 from the front side 100a to the rear side 100b, thereby forming the first locking loop 41 on the front side 100a (Fig. 3C), into the third through-hole 13 from the rear side 100b to the front side 100a, through the first locking loop 41 (Fig. 3D) and finally into the fourth through-hole 14 from the front side 100a to the back side 100b (Fig. 3E).

Thus, the first suspension section 231 extending between the implant G and the first through-hole 11 and the first end section 201 protruding from the fourth through-hole 14 are both arranged on the rear side 100b of the suspension plate 100, which allows to adjust the length of the first suspension section 231 from the rear side 100b by pulling on the first end section 201. This is advantageous for some applications of the implantable suspension device 1, where the rear side 100b of the suspension plate 100 can be more easily accessed than the front side 100a of the suspension plate 100.

The self-locking mechanism of the first locking loop 41 according to Fig. 3 functions in the same way as described above for the embodiment shown in Fig. 1 and the same forces act on the first suspension strand 231.

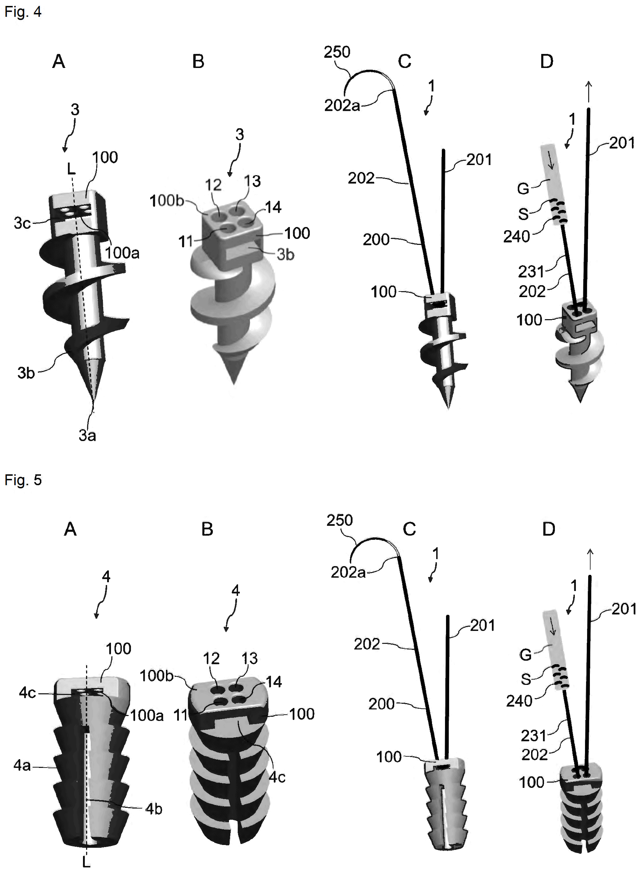

Fig. 4 and 5 show embodiments of the implantable suspension device 1, where the suspension plate 100 is integrally formed with a screw 3 (Fig. 4) or an anchor 4 (Fig. 5) for insertion into a bone.

The screw 3 shown in Fig. 4A extends along a longitudinal axis L between the suspension plate 100 and a pointed tip 3a, wherein the rear side 100b (see Fig. 4B) of the suspension plate 100 forms an end face of the screw 3 which is arranged perpendicular to the longitudinal axis L. The screw 3 further comprises a sharp cutting thread 3b configured to cut into a bone when the screw 3 screwed into the bone with the pointed tip 3a ahead.

The suspension plate 100 integrally formed with the screw 3 comprises a first through-hole 11, a second through-hole 12, a third through-hole 13 and a fourth through-hole 14, each extending between the front side 100a and the rear side 100b of the suspension plate 100. To access the front side 100a of the suspension plate 100, a lateral opening 3c is provided (Fig. 4A-B).

Fig. 5A shows an anchor 4 for insertion into a bore hole in a bone, which extends along a longitudinal axis L, wherein the rear side 100b (see Fig. 5B) of the suspension plate 100 forms an end face of the anchor 4 which is arranged perpendicular to the longitudinal axis L. The anchor 4 comprises a plurality of teeth 4a for engaging the inside walls of the bore hole in the bone to generate friction in order to tightly fix the anchor 4 in the bore hole. Furthermore, the anchor 4 comprises a groove 4b extending along the longitudinal axis L which groove 4b allows the teeth 4a to be pressed together during insertion into the bore hole.

The suspension plate 100 integrally formed with the anchor 4 comprises a first through-hole 11, a second through-hole 12, a third through-hole 13 and a fourth through-hole 14, each extending between the front side 100a and the rear side 100b of the suspension plate 100. To access the front side 100a of the suspension plate 100, a lateral opening 4c is provided (Fig. 5A-B).

As indicated in Fig. 4C and 5C, a suture element 200 can be inserted into the through-holes 11, 12, 13, 14 of the suspension plate 100, particularly in the same way as described for the embodiment of Fig. 3, thereby forming a locking loop for self-locking the suture element 200 on the suspension plate and resulting in a first suspension section 231 between a connecting section 240 attached to an implant G and the suspension plate 100 and a first end section 201 configured to be pulled to adjust the length of the first suspension section 231, wherein both the first suspension section 231 and the first end section 201 protrude from the same side (that is the rear side 100b) of the suspension plate 100 (Fig. 4C-D and 5C-D).

A needle 250 is attached to the second end 202a of the suture element 200 as shown in Fig. 4C and 5C to attach the connecting section 240 (positioned on the second end 202a) to the implant G by stitches S. Of course, the second end 202a of the suture element 200 may also be free, wherein the connecting section 240 is attached to the implant G e.g. by a separate needle. Furthermore, a needle 250 may also be attached to the second end 202a of the suture element 200 of the suspension devices 1 shown in Fig. 1, Fig. 2 or Fig. 3 in the same manner as illustrated in Fig. 4 and Fig. 5.

For example, the implantable suspension device 1 according to Fig. 4 and Fig. 5 may be used in the following manner: the screw 3 or anchor 4 is inserted into a bone with a dedicated screw driver or anchor driver, particularly with the first suspension section 231 and the first end section 201 embedded in a tube of the screw driver or anchor driver. Next, the implant G is stitched together with the connecting section 240 of the suture element 200. Finally, the first end section 201 is pulled to adjust the length of the first suspension section 231.

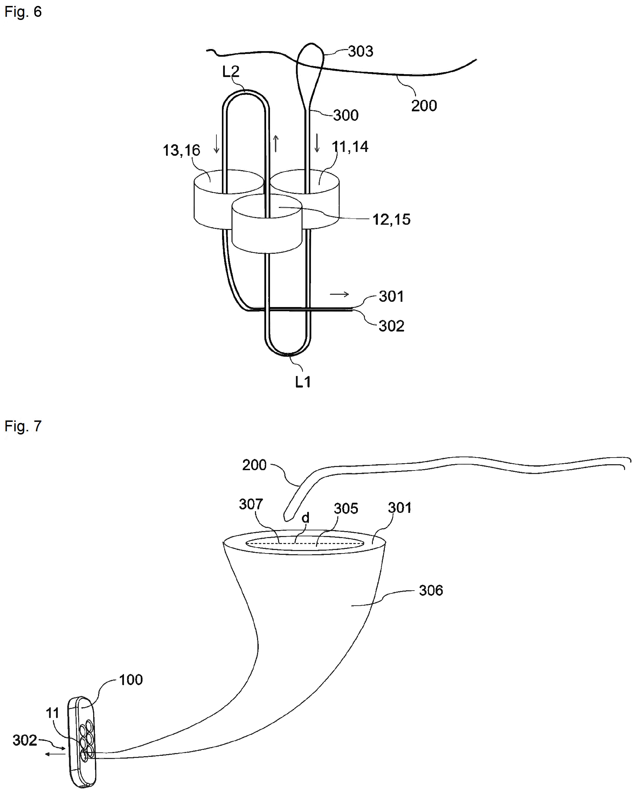

Fig. 6 schematically shows a method of assembling the suture element 200 with the suspension plate 100 to form the implantable suspension device 1 according to the invention. A guiding rope or guiding wire 300 is pre-assembled with the suspension plate 100 by inserting the guiding rope 300 into the through-holes 11, 12, 13, 14, 15, 16 of the suspension plate 100 in the same configuration as later desired for the suture element 200. In Fig. 6, three through-holes are schematically depicted, but of course the principle explained hereafter can also be applied to a suspension plate 100 with four or six through holes (see Fig. 2 and Fig. 3) within the scope of the invention.

The guiding rope 300 extends from a first end 301 successively through the third through-hole 13 or sixth through-hole 16, the second through-hole 12 or fifth through-hole 15, and the first through-hole 11 or fourth through-hole 14, forms a loop 303 and extends back through the first through-hole 11 or fourth through-hole 14, the second through-hole 12 or fifth through-hole 15 and the third through-hole 13 or the sixth through-hole 16 towards a second end 302. An additional first loop L1 or bend is formed between the first or fourth through-hole 11, 14 and the second or fifth through-hole 12, 15 and a further second loop L2 or bend is formed between the second or fifth through-hole 12, 15 and the third or sixth through-hole 13. 16.

When the suture element 200 is inserted into the loop 303 of the guiding rope 300 as shown in Fig. 6, the loop 303 may be successively advanced along the arrows depicted in Fig. 6 through the first or fourth through-hole 11, 14, the second or fifth through-hole 12, 15 and the third or sixth through-hole 13, 16 along with the suture element 200, resulting in the engagement of the suture element 200 with the suspension plate 100 shown in Fig. 1-3.

Fig. 7 depicts an alternative embodiment of the guiding rope 300, which comprises an inner volume 305 extending between a first end 301 and a second end 302 of the guiding rope 300. The guiding rope 300 is configured to receive the suture element 200 in the inner volume 305 and the guiding rope 300 forms a funnel 306 at the first end 301, the funnel 306 defining an opening 307 to receive the suture element 200 in the inner volume 305. In Fig. 7, an expanded state of the funnel 306 is shown, where the opening 207 has a diameter d. The funnel 306 is further characterized by a collapsed state, wherein the opening 307 has a diameter d which is smaller than in the depicted expanded state.

The guiding rope 300 according to Fig. 7 can be pre-assembled with the suspension plate 100 in the same manner as shown in Fig. 6, wherein the guiding rope 300 is inserted into the through-holes 11, 12, 13, 14, 15, 16 of the suspension plate 100 in the collapsed state. Then, the suture element 200 can be inserted into the inner volume 305 using the expanded state of the funnel 306 to easily insert the suture element 200 into the inner volume 305. To assemble the implantable suspension device 1, the guiding rope 300 can be moved together with the suture element 200 disposed in the inner volume 305 resulting in the suture element 200 engaging with the suspension plate 100 as explained above in relation to Fig. 6.

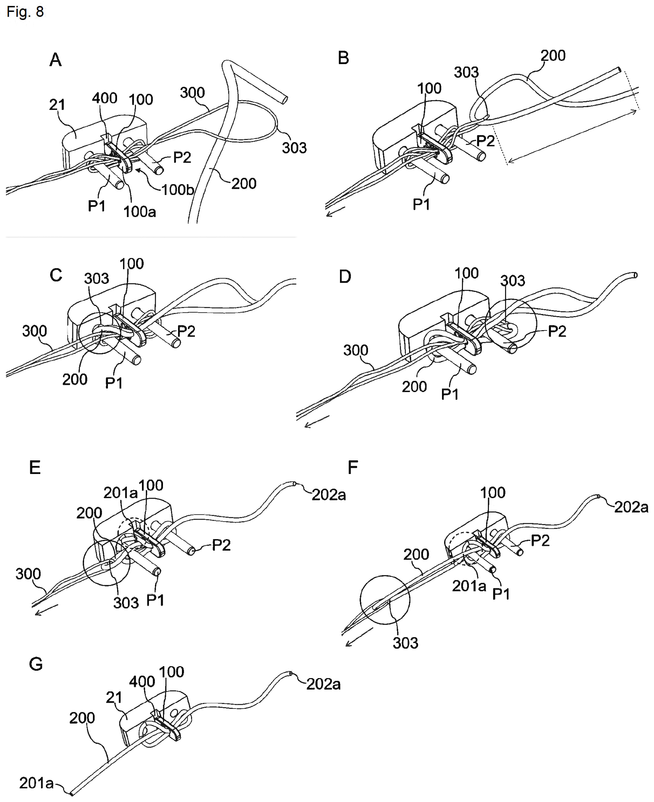

Hereafter, an assembly device 2 for assembling the implantable suspension device 1 according to the present invention is described with reference to Figures 8-13.

Fig. 8A-G show a holding part 21 of the assembly device 2 holding a suspension plate 100 with a pre-arranged guiding rope 300 as well as steps of assembling the suspension plate 100 with the suture element 200 using the assembly device 2 to form the implantable suspension device 1.

The holding part 21 comprises a holder 400 formed as a slot for insertion of the suspension plate 100, a first pin P1 and a second pin P2, wherein the first pin P1 and the second pin P2 are arranged on opposite sides of the holder 400. The holder 400 is arranged between the pins P1, P2 such that the first pin P1 faces the front side 100a of the suspension plate 100 and the second pin P2 faces the rear side 100b of the suspension plate 100. In Fig. 8, a suspension plate 100 with six through-holes 11, 12, 13, 14, 15, 16 is shown (see Fig. 2), but of course the assembly device 2 described here is also suitable for suspension plates with three (Fig. 1) or four (Fig. 3) through-holes 11, 12, 13, 14.

As shown in Fig. 8A, a guiding rope 300 extends through the first, the second and the third through-hole 11, 12, 13 in the same manner as schematically depicted in Fig. 6, and forms a loop 303 enlacing the suture element 200. Therein, the first loop L1 of the guiding wire 300 indicated in Fig. 6 is laid around the first pin P1 and the second loop L2 of the guiding wire 300 is laid around the second pin P2 to avoid entanglement of the guiding wire 300 and suture element 200 and avoid possible blocking of the assembly mechanism.

Fig. 8B-G show how the loop 303 of the guiding rope 300 is advanced through the through-holes of the suspension plate 100 along with the suture element 200 to pull the suture element 200 through the through-holes (in the same manner as schematically illustrated in Fig. 6) winding around the first pin P1 and the second pin P2, and resulting in engagement of the suture element 200 with the suspension plate 100 as shown in Fig. 1-3.

In Fig. 8C-F the loop 303 of the guiding rope 300 is marked with a solid circle and in Fig. 8E-F the first free end 201a of the suture element is marked with a dashed circle. As can be seen in Fig. 8F and 8G, after having passed through the first, the second and the third through-hole 11, 12, 13, the first end 201a of the suture element 200 slips through the loop 303 of the guiding rope 300 and detaches while being pulled.

Fig. 8G shows the final configuration of the suture element 200 in the suspension plate 100 held by the holder 400 of the holding part 21 after the guiding rope 300 has been removed. This setup is the same as the one shown in Fig. 2.

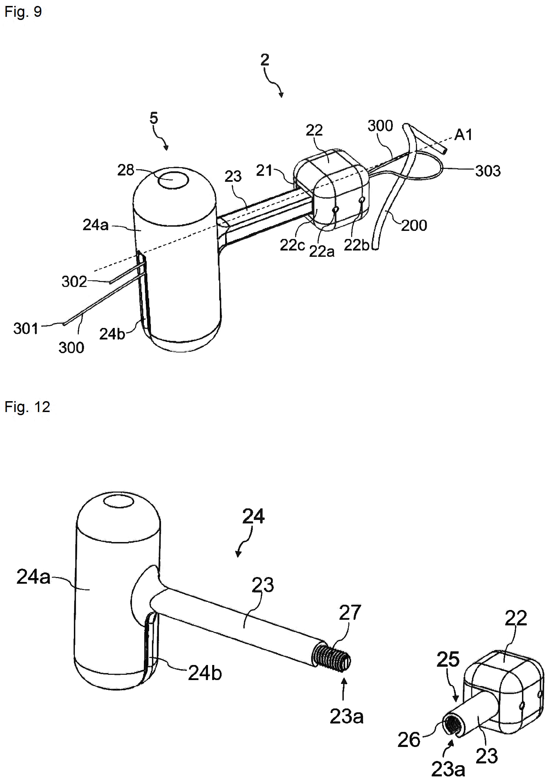

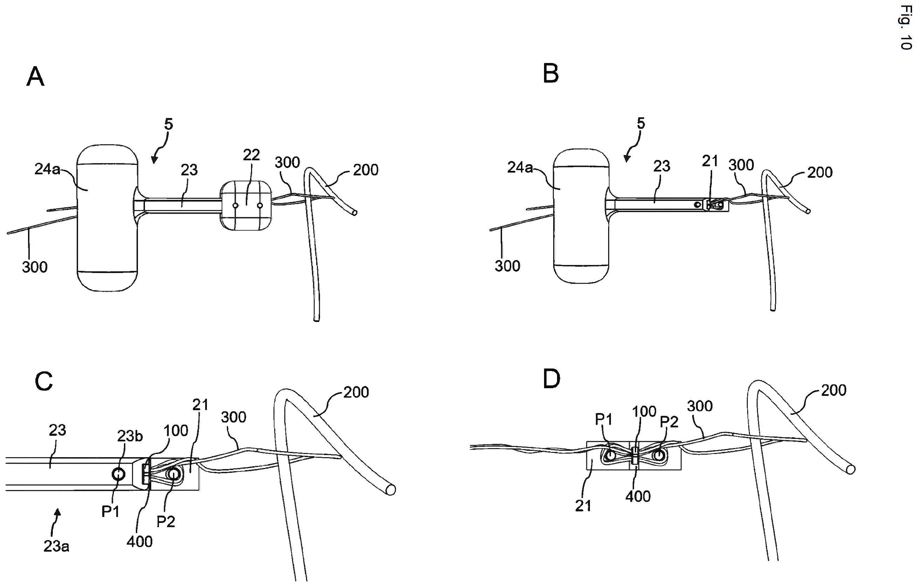

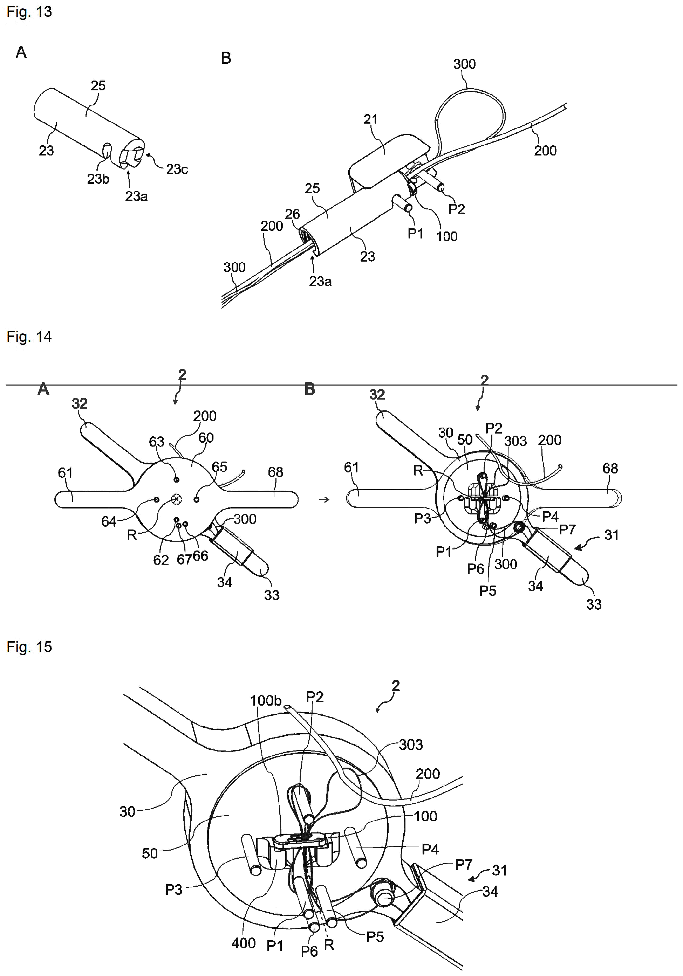

Fig. 9 shows an assembly device 2 comprising a holding part 21 (same as depicted in Fig. 8) assembled with a casing part 22 and a tool 5 for implanting the implantable suspension device 1 at a desired location in a human or animal body. The tool 5 comprises a guide bar 23 for guiding the suture element 200 and/or the guiding rope 300, extending along a first axis A1 and connected to the holding part 21 and the casing part 22, and a handle 24a for holding the tool 5, wherein the handle 24a is connected to the guide bar 23 at the opposite end in respect of the holding part 21 and the casing part 22. A guiding rope 300 forming a loop 303 that enlaces the suture element 200 extends along the first axis A1 through the casing part 22, along a slot 23a of the guide bar 23 (see Fig. 10C and 13A) and through a groove 24b of the handle 24a, the first end 301 and the second end 302 of the guiding rope 300 protruding from the groove 24b.

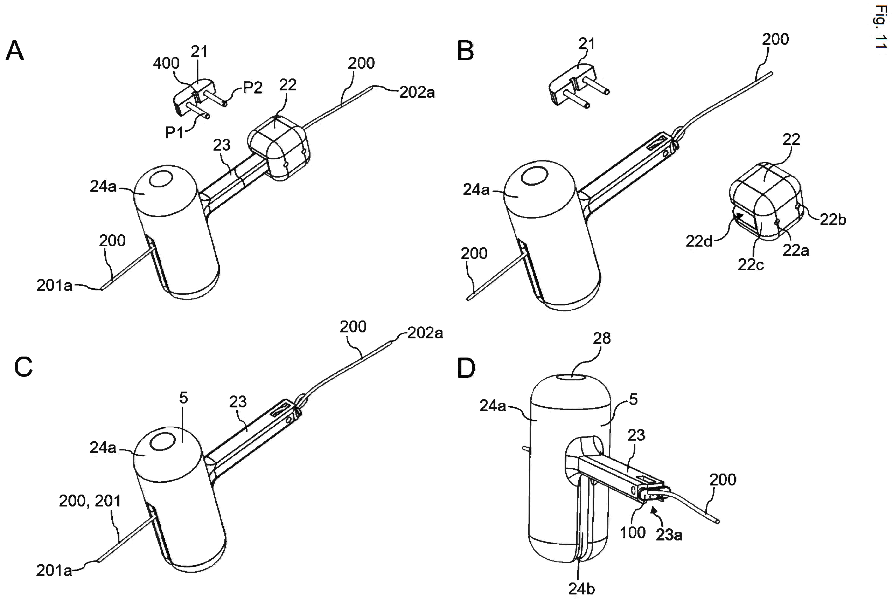

The casing part 22 comprises a first hole 22a and a second hole 22b for receiving the first pin P1 and the second pin P2 of the holding part 21, respectively, and connect the casing part 22 to the holding part 21, and a wall 22c encasing the holding part 21 and comprising an opening 22d for receiving the guide bar 23 (see also Fig. 11B for a detailed view).

Fig. 10A-D illustrate different components of the assembly device 2 shown in Fig. 9. Fig. 10B-D show the assembly device 2 without the casing part 22. As best seen in Fig. 10C, the guiding bar 23 comprises a hole or recess 23b for receiving the first pin P1 of the holding part 21 to connect the guiding bar 23 to the holding part 21. In addition, the guiding bar 23 may comprise a notch 23c positioned at the tip of the guiding bar 23 (see Fig. 13A) for inserting the suspension plate 100. Fig. 10D shows a side view of the holding part 21 of the assembly device 2 with the suspension plate 100 in the holder 400 and the guiding rope 300 extending through the through-holes 11, 12, 13, 14, 15, 16 of the suspension plate 100 and around the pins P1, P2 (see also Fig. 8A).

By pulling the guiding rope 300 through the assembly device 2 along the slot 23a of the guide bar 23 and through the groove 24b of the handle 24a, the suture element 200 may be assembled and engaged with the suspension plate 100 held by the holding part 21, resulting in the first end 201a of the suture element 200 protruding from the groove 24b of the handle 24a and the second end 202a of the suture element 200 protruding from the opening 22d of the casing part 22 opposite the tip of the guide bar 23, resulting in the setup shown in Fig. 11A when the holding part 21 is removed.

As depicted in Fig. 11B-D, the casing part 22 can then be removed from the tip of the guiding bar 23, leaving the suspension plate 100 with the suture element 200 held in the notch 23c at the tip of the guide bar 23.

The second end 202a of the suture element 200 may then be attached to an implant G (see Fig. 1-3) and the tool 5 may subsequently be used to place the suspension plate 100 at the desired location in the human or animal body using the handle 24a to grip the tool 5 and/or apply force to the suspension plate 100 if necessary. By pulling on the first end section 201 of the suture element 200 protruding from the groove 24b of the handle 24a, the length of the suspension section 231 can be adjusted as described above.

The tool 5 may further comprise an actuating element 28 that can be actuated to clamp the suture element 200 in the slot 23a of the guide bar 23 or release a clamping mechanism.

Fig. 12 and 13 show an embodiment of the assembly device 2 where the tool 5 is separable into a handle part 24 comprising the handle 24a and a first portion of the guiding bar 23 and a tip part 25 comprising a second portion of the guiding bar 23 which comprises the recess or hole 23b for receiving the first pin P1 of the holding part 21 and the notch 23c for inserting the suspension plate (see Fig. 13A-B).

As shown in Fig. 12, the tip part 25 comprises an inner thread 26 which corresponds to an outer thread 27 at the tip of the handle part 24, such that the outer thread 27 may be screwed into the inner thread 26 to assemble the handle part 24 and the tip part 25. In particular, the tip part 25 may be preassembled with the holding part 21, casing part 22, suspension plate 100 and guiding rope 300 as indicated in Fig. 12B and 13B, more particularly forming a disposable unit, whereas the handle part 24 of the tool 5 is reusable.

Fig. 14 and 15 illustrate a further embodiment of the assembly device 2 according to the present invention. The assembly device 2 comprises a first part 30 and a second part 50 which are rotatable in respect of each other about a rotation axis R (see Fig. 14A).

As depicted in Fig. 15, the second part 50 is formed as a circular plate arranged in a circular recess of the first part 30, such that the second part 50 can be rotated with respect to the first part 30 about the rotation axis R (which extends perpendicular to the circular plate at the center of the circular plate). The second part 50 comprises a holder 400 for holding the suspension plate 100, a first pin P1 and a second pin P2 arranged on opposite sides of the holder 400, such that the first pin P1 faces the front side 100a of the suspension plate 100 and the second pin P2 faces the rear side 100b of the suspension plate 100. The second part 50 further comprises a third pin P3 and a fourth pin P4 arranged at opposite sides of the holder 400 between the first pin P1 and the second pin P2, such that the first, second, third and fourth pin P1, P2, P3, P4 form the corners of an imaginary square along a periphery around the holder 400. A fifth pin P5 and a sixth pin P6 are arranged on the second part 50 adjacent the first pin P1 radially outside of the first pin P1.

The first part 30 comprises a fixing element 31 for fixing the guiding rope 300 to the first part 30. In the embodiment shown in Fig. 14 and 15, the fixing element 31 comprises a brace 34 for bracing or clamping the guiding rope 300 to the first part 30. A seventh pin P7 of the first part 30 is used to wind the guiding rope 300 around the seventh pin P7 and guide the guiding rope 300 to the fixing element 31.

As shown in Fig. 14A, the assembly device further comprises a lid part 60 comprising a first handle 61, a further handle 68 opposite the first handle 61, a first hole 62, a second hole 63 and further holes 64, 65, 66, 67 for receiving the first, second, third, fourth, fifth and sixth pin P1, P2, P3, P4, P5, P6 of the second part 50 (see Fig. 14B and 15) to couple the lid part 60 to the second part 50.

The first part 30 further comprises a second handle 32 and a further handle 33 opposite the second handle 32.

Fig. 14B and 15 further show a guiding rope 300 inserted into the through-holes 11, 12, 13, 14, 15, 16 of the suspension plate 100 as schematically depicted in Fig. 6 (with the first loop L1 around the first pin P1 and the second loop L2 around the second pin P2. The guiding rope 300 forms a loop 303 that enlaces a suture element 200, extends through three of the through-holes 11, 12, 13 of the suspension plate 100 in the holder 400 and extends between the fifth pin P5 and the sixth pin P6 towards the fixing element 31 on the first part 30.

When the lid part 60 is coupled to the second part 50 and the first handle 61 of the lid part 60 is moved relative to the second handle 32 of the first part 30, the second part 50 is rotated about the rotation axis R with respect to the first part 30. Thereby, the fixing element 31with the ends of the guiding rope 300 attached revolves around the second part 50 and the loop 303 of the guiding rope 300 is drawn around the pins P1, P2 and through the through-holes 11, 12, 13 of the suspension plate 100 in the holder 400, resulting in an engagement of the suture element 200 with the suspension plate 100 similar to the procedure shown in Fig. 8A-G.

Due to the rotary motion of the guiding rope 300, the assembly advantageously requires less space than in case of a linear motion. In addition, uncontrollable movements during the mechanism due to different forces acting during different stages of assembly, are avoided by the rotary assembly procedure. Furthermore, by using sufficiently long handles, the force applied to the handles can be amplified (e.g. by a factor of about 3), resulting in less force applied by the user. The pins P1, P2, P3, P4 ensure that the guiding rope 300 and the suture element 200 do not entangle and the pins P5, P6, P7 serve to guide the guiding rope towards the fixing element 31.

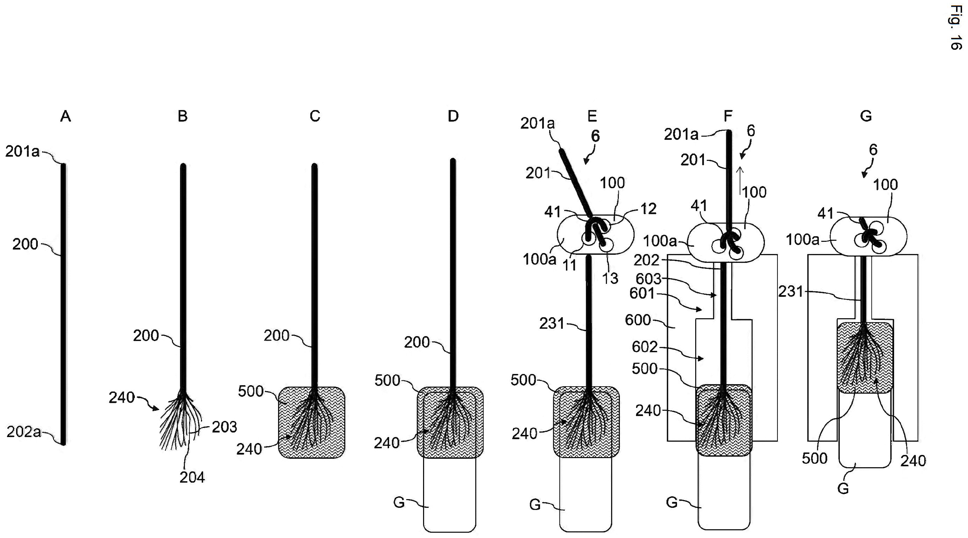

Fig. 16 A-G show assembly steps of a medical implant 6 according to an embodiment of the invention comprising a medical textile 500, e.g. from a felt material, and an implantable suspension device 1 according to the invention which comprises a suspension plate 100 and a suture element 200 comprising a first end 201a and a second end 202a (Fig. 16A).

As shown in Fig. 16B, the suture element 200 comprises at the second end 202a, a connecting section 240 composed of separated fibers 203, each comprising a fiber end 204. The fibers 203 are spread out in a fanlike manner, thereby increasing the area of the connecting section 240. In particular, the entire suture element 200 is formed from braided or twisted fibers 203, which have been separated from each other at the second end 202a to form the connecting section 240.

Next, as illustrated in Fig. 16C, the connecting section 240 of the suture element 200 is connected to a medical textile 500 in form of a patch. In particular, to connect the medical textile 500 to the connecting section 240, the medical textile 500 and the connecting section 240 are placed on top of each other and a felting needle comprising at least one barb is repeatedly advanced through the connecting section 240 and the medical textile 500. Thereby, the fibers 203 of the connecting section 240 are interwoven with the material of the medical textile 502, resulting in a strong connection. In case the medical textile 500 is formed from a felt material, advancing the felting needle through the connecting section 240 and the medical textile 500 also results in pulling out fibers from the felt material of the medical textile 500, which further contributes to the connection.

Subsequently, the medical textile 500, which is now attached to the suture element 200, is connected to a flexible implant G, e.g., a soft tissue such as a tendon (Fig. 16D). This is particularly achieved by placing the medical textile 500 onto the flexible implant G and repeatedly advancing a felting needle having at least one barb through the medical textile 500 into the flexible implant G, which results in pulling out fibers from the material, e.g., the felt material, of the medical textile 500 (and particularly also fibers 203 of the connecting section 240 of the medical suture 200) and inserting these fibers into the flexible implant G. Thereby, a strong connection between the flexible implant G and the medical textile 500, as well as the attached suture element 200 can be obtained in a relatively easy manner.

Fig. 16 E illustrates the assembly of the suture element 200 with the suspension plate 100 to form the medical implant 6, which includes an implantable suspension device 1 composed of the suture element 200 and the suspension plate 100. According to this example, the suspension plate 100 comprises three through holes 11, 12, 13, which are arranged as in the example of the implantable suspension device 1 shown in Fig. 1. The first end 201a of the suture element 200 is advanced through the through holes 11, 12, 13, and a first locking loop 41 is formed as described above and illustrated in Fig. 1. Furthermore, as described above, the section of the suture element 200 between the suspension plate 100 and the connecting element 200/medical textile 500 forms a first suspension section 231, the length of which can be adjusted by pulling on the first end section 201.

Finally, Fig. 16F and 16G show the insertion of the medical implant 6 into a bone tunnel 601 of a bone 600 to fix the flexible implant G on the bone 600, e.g. during anterior cruciate ligament (ACL) repair. The bone tunnel 601 comprises a first segment 602 and an adjacent second segment 603 with a smaller diameter compared to the first segment 602. The diameter of the first segment 602 particularly matches the approximate width of the flexible implant G, and the second segment 603 has dimensions allowing the suspension plate 100 to be moved through the second segment 603.

During a typical surgical procedure, the bone tunnel 601 is first introduced into the bone 600 using a surgical drill. Subsequently, the suspension plate 100 with the attached suture element 200 is introduced into the first segment 602 of the bone tunnel 601 and then moved through the second segment 603 of the bone tunnel 601 to the outside of the bone 600. The suspension plate 100 is then flipped to be arranged perpendicular to the bone tunnel 601. Next, the first suspension section 231 is shortened by pulling on the first end section 201, which results in the medical textile 500 with the attached flexible implant G being pulled into the first segment 602 of the bone tunnel 601. After tightening the first suspension section 231, the first end section 201 of this is suture element 200 may be cut, resulting in the configuration shown in Fig. 16 G.

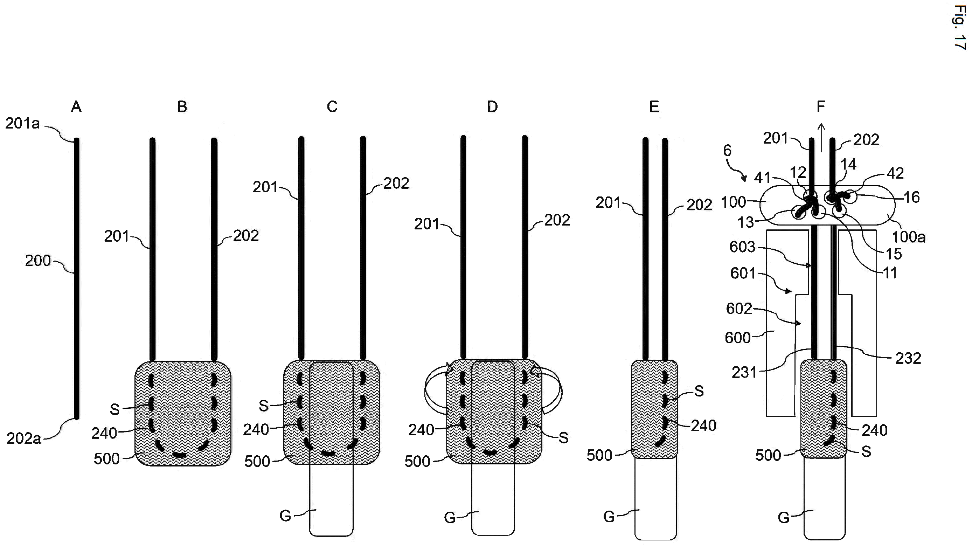

Fig. 17 A-F show assembly steps of a medical implant 6 according to a further embodiment of the invention comprising a medical textile 500, e.g. from a felt material, and an implantable suspension device 1 according to the invention which comprises a suspension plate 100 and a suture element 200 comprising a first end 201a and a second end 202a (Fig. 17A).

As shown in Fig. 17 B, a central connecting section 240 of the suture element 200 is connected to a medical textile 500, e.g. a patch from a felt material, by stitches S, resulting in a first end section 201 and a second end section 202 protruding from the medical textile 500. The stitch S connection of the suture element 200 and the medical textile 500 results in an improved tensile strength of the medical textile 500.

Fig. 17 C to Fig. 17 E illustrate how a flexible implant G is connected to the medical textile 500 according to an example of the invention. As shown in Fig. 17 C, the medical textile 500 with the attached suture element 200 is first placed on the flexible implant G. Next, the medical textile 500 is at least partially wrapped around the flexible implant G as indicated by the arrows in Fig. 17 D, thereby forming a tubular structure which is arranged around a part of the flexible implant G (Fig. 17 E). Subsequently, the medical textile 500 is connected to the flexible implant G, in particular, by repeatedly advancing a felting needle comprising at least one barb through the medical textile 500 and the flexible implant G, which results in fibers of the medical textile 500 being inserted into the flexible implant G. Optionally, the medical textile 500 may additionally be pre-attached to the flexible implant G, e.g. by repeatedly advancing a felting needle through the assembly shown in Fig. 17 C.

Finally, as illustrated in fig. 17 F, a suspension plate 100 having six through holes 11, 12, 13, 14, 15, 16 is assembled with the first end section 201 and the second end section 202 of the suture element 200, and a first locking loop 41 and a second locking loop 42 are formed in a similar manner as described above and shown in Fig. 2.

The assembled medical implant 6 is then inserted into a bone tunnel 601 as described above for the example shown in Fig. 16, and the first suspension section 231 and second suspension section 232 formed between the suspension plate 100 and the medical textile 500 /connecting section 240 are shortened by pulling on the first end section 201 and the second end section 202 (Fig. 17G).

In the following, aspects of the present invention and embodiments thereof are stated as items, wherein the reference numerals in parentheses refer to the Figures. These items may also be formulated as claims of the present invention:
Item1: An implantable suspension device (1) for fixing an elongated flexible implant (G) in a desired position, comprising: a suspension plate (100) and a suture element (200) engaging with the suspension plate (100), wherein the suture element (200) comprises a first end section (201) extending from said suspension plate (100) to a first end (201a) of said suture element (200) and a second end section (202) extending from said suspension plate (100) to a second end (202a) of said suture element (200), wherein the second end section (202) comprises a connecting section (240) configured to be connected to said implant (G) or to a medical textile (500).
Item 2: The implantable suspension device (1) according to item 1, wherein said connecting section (240) is positioned at the second end (202a) of the suture element (200).
Item 3: The implantable suspension device (1) according to item 1 or 2, wherein the suture element (200) comprises a needle (250) attached to the suture element (200), particularly to the first end (201a) or the second end (202a).
Item 4: The implantable suspension device (1) according to item 1 or 2, wherein said suture element (200) comprises or consists of a plurality of fibers (203), wherein the fibers (203) are separated from each other in the connecting section (240), particularly wherein each of the separated fibers (203) comprises a fiber end (204) or a fiber loop positioned at the second end (202a) of the suture element (200).
Item 5: The implantable suspension device (1) according to item 4, wherein said connecting section (240) forms a fan-like structure extending in a plane parallel to an extension direction of the suture element (200).
Item 6: The implantable suspension device (1) according to one of the preceding items, wherein the suture element (200) forms a first locking loop (41) for pressing the first end section (201) of the suture element (200) against the suspension plate (100) and thereby locking the first end section (201) with respect to the suspension plate (100).
Item 7: The implantable suspension device (1) according to one of the preceding items, wherein the second end section (202) comprises an adjustable first suspension section (231) between said suspension plate (100) and said connecting section (240), wherein the first suspension section (231) is configured to be shortened by pulling on the first end section (201) of the suture element (200), wherein particularly said first end section (201) protrudes out of said first locking loop (41).
Item 8: The implantable suspension device (1) according to one of the preceding items, wherein the suspension plate (100) comprises a front side (100a) and a rear side (100b), which rear side (100b) faces away from the front side (100a).
Item 9: The implantable suspension device (1) according to item 8, wherein the suspension plate (100) comprises a plurality of through-holes (11, 12, 13, 14, 15, 16), each through-hole (11, 12, 13, 14, 15, 16) extending from the front side (100a) to the rear side (100b) of said suspension plate (100), wherein said suture element (200) extends through said through-holes (11, 12, 13, 14, 15, 16).
Item 10: The implantable suspension device (1) according to item 9, wherein said plurality of through-holes (11, 12, 13, 14, 15, 16) is formed by at least three through-holes (11, 12, 13), particularly 3, 4, or 6 through-holes.
Item 11: The implantable suspension device (1) according to item 9 or 10, wherein the suture element (200) extends through a first-through-hole (11) of said plurality of through-holes (11, 12, 13, 14, 15, 16) from the rear side (100b) to the front side (100a) of the suspension plate (100), with the first end (201a) ahead, particularly such that the adjustable first suspension section (231) is formed on the rear side (100b) of the suspension plate (100).
Item 12: The implantable suspension device (1) according to item 11, wherein the suture element (200) extends through a second through-hole (12) of said plurality of through-holes from the front side (100a) to the rear side (100b) of the suspension plate (100), with the first end (201a) ahead, particularly such that the first locking loop (41) is formed on the front side (100a) of the suspension plate (100), wherein the suture element (200) further extends through a third through-hole (13) of said plurality of through-holes (11, 12, 13, 14, 15, 16) from the rear side (100b) to the front side (100a) of the suspension plate (100) with the first end (201a) ahead, wherein particularly the first end section (201) of the suture element (200) further extends through the first locking loop (41) for clamping the first end section (201) to the front side (100a) of the suspension plate (100) by means of the first locking loop (41) with a locking force (F_{L}).
Item 13: The implantable suspension device (1) according to item 12, wherein the suture element (200) further extends through a fourth through-hole (14) of said plurality of through-holes from the front side (100a) to the rear side (100b) of the suspension plate (100) with the first end (201a) ahead.
Item 14: The implantable suspension device (1) according to item 12, wherein the suture element (200) further extends through a fourth through-hole (14) of said plurality of through-holes (11, 12, 13, 14, 15, 16) from the rear side (100b) to the front side (100a) of the suspension plate (100) with the second end (202a) ahead, particularly such that the suture element (200) forms a second suspension section (232) between said connecting section (240) and said suspension plate (100), wherein said suture element (200) further extends through a fifth through-hole (15) of said plurality of through-holes (11, 12, 13, 14, 15, 16) from the front side (100a) to the rear side (100b) of the suspension plate (100) with the second end (202a) ahead, particularly such that a second locking loop (42) is formed, wherein the suture element (200) further extends through a sixth through-hole (16) of said plurality of through-holes (11, 12, 13, 14, 15, 16) from the rear side (100b) to the front side (100a) of the suspension plate (100) with the second end (202a) ahead, wherein particularly the second end section (202) of the suture element (200) further extends through said second locking loop (42) for clamping the second end section (202) of the suture element (200) to the front side (100a) of the suspension plate (100) by means of the second locking loop (42) with a locking force (F_{L}).
Item 15: The implantable suspension device (1) according to item 14, wherein the suture element (200) comprises an adjustable second suspension section (232) between said connecting section (240) and said suspension plate (100), wherein the second suspension section (232) is configured to be shortened by pulling on the second end section (202) of the suture element (200), wherein particularly said second end section (202) protrudes out of said second locking loop (42).
Item 16: The implantable suspension device (1) according to one of the preceding items, wherein the suspension plate (100) is connected to or integrally formed with a screw (3) or an anchor (4) configured to be inserted into a bone.
Item 17: A medical implant (6) comprising the implantable suspension device (1) according to one of the items 1 to 16 and a medical textile (500), particularly comprising or consisting of a felt material, wherein the connecting section (240) of the suture element (200) is connected to the medical textile (500), wherein the medical textile (500) is configured to be connected to said implant (G).
Item 18: The medical implant (6) according to item 17, wherein the connecting section (240) is connected to the medical textile (500) by stitches (S).
Item 19: The medical implant (6) according to item 17, wherein the suture element (200) comprises or consists of a plurality of fibers (203), wherein the fibers (203) are separated from each other in the connecting section (240), and wherein the separated fibers (203) of the connecting section (240) extend into and/or through the medical textile (500) to connect the suture element (200) of the implantable suspension device (1) to the medical textile (300).
Item 20: The medical implant (6) according to item 19, wherein the medical textile (500) extends along a plane, wherein particularly the medical textile (500) comprises a flat shape, wherein particularly the connecting section (240) of the suture element (200) forms a fan-like structure extending in a plane parallel to an extension direction of the suture element (200), and wherein the suture element (200) is connected to the medical textile (500), such that the first medical implant (130) extends from the medical textile (500) parallel to the plane of the medical textile (500).
Item 21: The medical implant (6) according to one of the items 17 to 19, wherein the medical textile (500) comprises a tubular shape, wherein the medical textile (500) comprises an inner surface configured to be connected to said implant (G).
Item 22: An assembly device (2) for assembling the implantable suspension device (1) according to one of the items 1 to 16, comprising
   - a holder (400) configured to hold said suspension plate (100),
   - a guiding rope (300) configured to be moved together with said suture element (200) relative to the holder (400) resulting in the suture element (200) engaging with the suspension plate (100) to assemble said implantable suspension device (1).
Item 23: The assembly device (2) according to item 22, wherein the assembly device (2) is configured to assemble the implantable suspension device (1) according to any one of the items 6 to 14, wherein said guiding rope (300) extends through at least one through-hole (11, 12, 13, 14, 15, 16) of said plurality of through-holes (11, 12, 13, 14, 15, 16) of said suspension plate (100) held by said holder (400), such that the guiding rope (300) can be moved through said at least one through-hole (11, 12, 13, 14, 15, 16) together with the suture element (200), resulting in the suture element (200) engaging with the suspension plate (100).
Item 24: The assembly device (2) according to item 22 or 23, wherein said assembly device (2) comprises a first pin (P1) and a second pin (P2), wherein said holder (400) is configured to hold the suspension plate (100) between said first pin (P1) and said second pin (P2), and wherein the assembly device (2) is configured such that the guiding rope (300) can be moved around the first pin (P1) and the second pin (P2) together with the suture element (200) resulting in the suture element (200) engaging with the suspension plate (100), particularly wherein the holder (400) is configured to hold the suspension plate (100) between the first pin (P1) and the second pin (P2) such that said first pin (P1) faces said front side (100a) of the suspension plate (100) and said second pin (P2) faces said rear side (100b) of the suspension plate (100).
Item 25: The assembly device (2) according to item 23 or 24 when referring to item 16, wherein the assembly device is configured to assemble the implantable suspension device (1) according to one of the items 9 to 12 or item 13 or 14 when referring to one of the items 9 to 12, wherein said guiding rope (300) extends successively through said first through-hole (11) or said fourth through-hole (14) from said rear side (100b) to said front side (100a) of the suspension plate (100), around said first pin (P1), through said second through-hole (12) or said fifth through-hole (15) from said front side (100a) to said rear side (100b) of the suspension plate (100), around said second pin (P2) and through said third through-hole (13) or said sixth through-hole (16) from said rear side (100b) to said front side (100a) of the suspension plate (100).
Item 26: The assembly device (2) according to one of the items 22 to 25, wherein said guiding rope (300) forms a loop (303) configured to enlace said suture element (200), such that said loop (303) can be moved together with the suture element (200) relative to the holder (400) resulting in the suture element (200) engaging with the suspension plate (100).
Item 27: The assembly device (2) according to one of the items 22 to 26, wherein said guiding rope (300) comprises an inner volume (305) extending between a first end (301) and a second end (302) of the guiding rope (300), wherein the guiding rope (300) is configured to receive said suture element (200) in said inner volume (305), such that the guiding rope (300) can be moved together with the suture element (200) disposed in the inner volume (305) relative to the holder (400) resulting in the suture element (200) engaging with the suspension plate (100), wherein particularly said guiding rope (300) forms a funnel (306) at the first end, wherein said funnel (306) defines an opening (307) for receiving said suture element (200) in said inner volume (305), wherein particularly the funnel (306) is characterized by an expanded state and a collapsed state, wherein said opening (307) has a first diameter in the expanded state and a second diameter in the collapsed state, wherein the first diameter is larger than the second diameter.
Item 28: The assembly device (2) according to one of the items 22 to 27, wherein the assembly device (2) comprises a holding part (21) comprising said holder (400) and a casing part (22) comprising a wall (22c) configured to encase said holder (400) when the holding part (21) and the casing part (22) are assembled, wherein particularly the wall (22c) comprises an opening (22d) for inserting and/or extracting the suture element (200) by means of the guiding rope (300), particularly the first end (201a) and/or the second end (202a) of the suture element (200), wherein particularly said holding part (21) comprises said first pin (P1) and said second pin (P2), wherein said casing part comprises a first hole (22a) configured to receive the first pin (P1) of the holding part (21) and a second hole (22b) configured to receive the second pin (P2) of the holding part (21) to assemble the holding part (21) and the casing part (22).
Item 29: The assembly device (2) according to one of the items 22 to 28, wherein the assembly device (2) comprises a tool (5) for implanting said implantable suspension device (1), wherein the tool (5) comprises a guide bar (23) extending along a first axis (A1), wherein the guide bar (23) comprises a slot (23a) extending along said first axis (A1) for receiving said guiding rope (300) and/or said suture element (200), particularly said first end (201a) or said second end (202a) of said suture element (200).
Item 30: The assembly device (2) according to item 29 and item 24 or one of the items 25 to 28 when referring to item 24, wherein said guide bar (23) comprises a recess (23b) for receiving said first pin (P1) or said second pin (P2).
Item 31: The assembly device (2) according to item 30 or item 29 and item 24 or one of the items 25 to 28 when referring to item 24, wherein said guide bar (23) comprises a tip (23d) comprising a notch (23c) for receiving said suspension plate (100).
Item 32: The assembly device (2) according to item 30 or 31, wherein said tool (5) comprises a handle (24a) for holding said tool (5), wherein said handle (24a) is connected to said guide bar (23), wherein particularly said handle (24a) comprises a groove (24b) for inserting said guiding rope (300) and/or said suture element (200) when at least a part of said guiding rope (300) and/or said suture element (200) is arranged in the slot (23a).
Item 33: The assembly device (2) according to item 32, wherein the tool (5) is separable into a handle part (24) comprising said handle (24a) and a tip part (25) comprising at least a part of said guiding bar (23), wherein particularly said handle part (24) and said tip part (25) comprise an inner thread (26) and an outer thread (27) corresponding to the inner thread (26).
Item 34: The assembly device (2) according to one of the items 30 to 33, wherein said assembly device (2) is configured to clamp the suture element (200) in the slot (23a).
Item 35: The assembly device (2) according to item 34, wherein said assembly device (2) comprises an actuating element (28) that can be actuated to clamp the suture element (200) in the slot (23a).
Item 36: The assembly device (2) according to item 34, wherein said assembly device (2) comprises a self-locking clamp for clamping the suture element (200) when the suture element (200) is received in the slot (23a).
Item 37: The assembly device (2) according to one of the items 22 to 27, wherein said assembly device comprises a first part (30) comprising a fixing element (31) for attaching the guiding rope (300) to the first part (30), and a second part (50) comprising said holder (400), wherein said second part (50) is rotatable about a rotation axis (R) relative to said first part (30), such that the guiding rope (300) can be moved together with said suture element (200) relative to the holder (400) when said second part (50) is rotated relative to said first part (30), resulting in the suture element (200) engaging with the suspension plate (100).
Item 38: The assembly device (2) according to item 37 and item 24 or one of the items 25 to 27 when referring to item 24, wherein said first pin (P1) and said second pin (P2) are comprised in said second part (50), wherein said assembly device (2) further comprises a third pin (P3) and a fourth pin (P4) comprised in said second part (50), the third pin (P3) being arranged between the first pin (P1) and the second pin (P2) along a periphery around the holder (400) and the fourth pin (P4) being arranged between the first pin (P1) and the second pin (P2) and opposite the third pin (P3) along said periphery, wherein said guiding rope (300) is arrangeable along said periphery around said first pin (P1), said second pin (P2), said third pin (P3) and said fourth pin (P4) when said second part (50) is rotated relative to said first part (30).
Item 39: The assembly device (2) according to item 38, wherein said assembly device (2) further comprises a fifth pin (P5) for guiding said guiding rope (300) and/or said suture element (200) from the holder (400) towards the fixing element (31), wherein the fifth pin (P5) is arranged on said second part (50) adjacent to said first pin (P1) radially outward from said first pin (P1) with respect to said rotation axis (R).
Item 40: The assembly device (2) according to item 39, wherein said assembly device (2) further comprises a sixth pin (P6) arranged on said second part (50), wherein the fifth pin (P5) and the sixth pin (P6) are arranged such that the guiding rope (300) and/or the suture element (200) can be guided from the holder (400) towards the fixing element (31) between the fifth pin (P5) and the sixth pin (P6) when said second part (50) is rotated relative to said first part (30).
Item 41: The assembly device (2) according to one of the items 37 to 40, wherein said assembly device (2) comprises a lid part (60) comprising a first handle (61), wherein the lid part (60) is configured to be coupled to said second part (50), wherein said first part (30) comprises a second handle (32), such that the second part (50) can be rotated relative to said first part (30) by moving said first handle (61) relative to said second handle (32), wherein particularly said lid part (60) comprises at least a first hole (62) and a second hole (63), wherein the first hole (62) and the second hole (63) are each configured to receive one of said first pin (P1), said second pin (P2), said third pin (P3), said fourth pin (P4), said fifth pin (P5) or said sixth pin (P6) to couple the lid part (60) to the second part (50).
Item 42: A method for assembling an implantable suspension device (1), particularly according to one of the items 1 to 16, wherein a suspension plate (100) and a suture element (200) are provided, wherein the suture element (200) is engaged with the suspension plate (100), such that a first end section (201) of the suture element (200) extends from said suspension plate (100) to a first end (201a) of said suture element (200) and a second end section (202) extends from said suspension plate (100) to a second end (202a) of said suture element (200).
Item 43: The method according to item 42, wherein a connecting section (240) comprised in the second end section (202) is connected, particularly stitched, to an elongated flexible implant (G).
Item 44: The method according to item 42 or 43, wherein a first locking loop (41) is formed from the suture element (200), wherein the first locking loop (41) presses the first end section (201) of the suture element (200) against the suspension plate (100), thereby locking the first end section (201) with respect to the suspension plate (100).
Item 45: The method according to one of the items 42 to 44, wherein an adjustable first suspension section (231) of said suture element (200) between said suspension plate (100) and said connecting section (240) is shortened by pulling on the first end section (201) of the suture element (200), wherein particularly said first end section (201) protrudes out of said first locking loop (41).
Item 46: The method according to one of the items 42 to 45, wherein said suture element (200) is inserted into a plurality of through-holes (11, 12, 13, 14, 15, 16) of said suspension plate (100), particularly at least three through-holes (11, 12, 13), more particularly 3, 4 or 6 through-holes, extending from a front side (100a) of the suspension plate (100) to a rear side (100b) of the suspension plate (100) facing away from the front side (100a).
Item 47: The method according to item 46, wherein the suture element (200) is inserted into a first-through-hole (11) of said plurality of through-holes (11, 12, 13, 14, 15, 16) from the rear side (100b) to the front side (100a) of the suspension plate (100) with the first end (201a) ahead, particularly such that the adjustable first suspension section (231) is formed on the rear side (100b) of the suspension plate (100).
Item 48: The method according to item 47, wherein the suture element (200) is inserted into a second through-hole (12) of said plurality of through-holes from the front side (100a) to the rear side (100b) of the suspension plate (100) with the first end (201a) ahead, particularly such that the first locking loop (41) is formed on the front side (100a) of the suspension plate (100), wherein the suture element (200) is further inserted into a third through-hole (13) of said plurality of through-holes (11, 12, 13, 14, 15, 16) from the rear side (100b) to the front side (100a) of the suspension plate (100) with the first end (201a) ahead, wherein particularly the first end section (201) of the suture element (200) is further inserted into the first locking loop (41) for clamping the first end section (201) to the front side (100a) of the suspension plate (100) by means of the first locking loop (41) with a locking force (F_{L}).
Item 49: The method according to item 48, wherein the suture element (200) is further inserted into a fourth through-hole (14) of said plurality of through-holes from the front side (100a) to the rear side (100b) of the suspension plate (100) with the first end (201a) ahead.
Item 50: The method according to item 48, wherein the suture element (200) is further inserted into a fourth through-hole (14) of said plurality of through-holes (11, 12, 13, 14, 15, 16) from the rear side (100b) to the front side (100a) of the suspension plate (100) with the second end (202a) ahead, particularly such that the suture element (200) forms a second suspension section (232) between said connecting section (240) and said suspension plate (100), wherein said suture element (200) is further inserted into a fifth through-hole (15) of said plurality of through-holes (11, 12, 13, 14, 15, 16) from the front side (100a) to the rear side (100b) of the suspension plate (100) with the second end (202a) ahead, particularly such that a second locking loop (42) is formed, wherein the suture element (200) is further inserted into a sixth through-hole (16) of said plurality of through-holes (11, 12, 13, 14, 15, 16) from the rear side (100b) to the front side (100a) of the suspension plate (100) with the second end (202a) ahead, wherein particularly the second end section (202) of the suture element (200) is further inserted into said second locking loop (42) for clamping the second end section (202) of the suture element (200) to the front side (100a) of the suspension plate (100) by means of the second locking loop (42) with a locking force (F_{L}).
Item 51: The method according to item 50, wherein an adjustable second suspension section (232) of said suture element (200) between said connecting section (240) and said suspension plate (100) is shortened by pulling on the second end section (202) of the suture element (200), wherein particularly said second end section (202) protrudes out of said second locking loop (42).
Item 52: The method according to one of the items 42 to 51, wherein the connecting section (240) is connected to the elongated flexible implant (G) by means of a needle (250) attached to the suture element (200), particularly to the first end (201a) or the second end (202a).
Item 53: The method according to one of the items 42 to 52, wherein a guiding rope (300) is moved together with said suture element (200) resulting in the suture element (200) engaging with the suspension plate (100) to assemble said implantable suspension device (1).
Item 54: The method according to item 53 when referring to one of the items 45 to 51, wherein said guiding rope (300) is inserted into at least one through-hole (11, 12, 13, 14, 15, 16) of said plurality of through-holes (11, 12, 13, 14, 15, 16) of said suspension plate (100), and wherein the guiding rope (300) is subsequently moved through said at least one through-hole (11, 12, 13, 14, 15, 16) together with the suture element (200), resulting in the suture element (200) engaging with the suspension plate (100).
Item 55: The method according to item 53 or 54, wherein the guiding rope (300) is moved around a first pin (P1) and a second pin (P2) together with the suture element (200) during engagement of the suture element (200) with the suspension plate (100), particularly wherein the suspension plate (100) is arranged between the first pin (P1) and the second pin (P2), such that said first pin (P1) faces said front side (100a) of the suspension plate (100) and said second pin (P2) faces said rear side (100b) of the suspension plate (100).
Item 56: The method according to one of the items 53 to 55, wherein said guiding rope (300) is successively inserted into said first through-hole (11) or said fourth through-hole (14) from said rear side (100b) to said front side (100a) of the suspension plate (100), around said first pin (P1), into said second through-hole (12) or said fifth through-hole (15) from said front side (100a) to said rear side (100b) of the suspension plate (100), around said second pin (P2) and into said third through-hole (13) or said sixth through-hole (16) from said rear side (100b) to said front side (100a) of the suspension plate (100).
Item 57: The method according to one of the items 53 to 56, wherein said guiding rope (300) forms a loop (303) enlacing said suture element (200), wherein said loop (303) is moved together with the suture element (200) resulting in the suture element (200) engaging with the suspension plate (100).
Item 58: The method according to one of the items 53 to 57, wherein said suture element (200) is received in an inner volume (305) of the guiding rope (300) extending between a first end (301) and a second end (302) of the guiding rope (300), wherein the guiding rope (300) is moved together with the suture element (200) disposed in the inner volume (305) resulting in the suture element (200) engaging with the suspension plate (100), wherein particularly said suture element (200) is received in said inner volume (305) through an opening (307) of a funnel (306) formed by the guiding rope (300) at the first end (301) of the guiding rope (300), wherein particularly the funnel (306) is collapsed from an expanded state to a collapsed state during engagement of the suture element (200) with the suspension plate (100), wherein said opening (307) has a first diameter in the expanded state and a second diameter in the collapsed state, wherein the first diameter is larger than the second diameter.
Item 59: The method according to one of the items 42 to 58, wherein after assembly of said implantable suspension device (1), said implantable suspension device (1) is mounted, particularly automatically, on a tool (5) for implanting said implantable suspension device (1). Item 60: The method according to one of the items 53 to 59, wherein the guiding rope (300) is attached to a fixing element (31), wherein the fixing element (31) is moved along a periphery around the suspension plate (100), such that the guiding rope (300) together with the suture element (200) is moved relative to the suspension plate (100) resulting in the suture element (200) engaging with the suspension plate (100).
Item 61: The method according to item 60 and item 55 or one of the items 56 to 59 when referring to item 55, wherein said guiding rope (300) is moved along said periphery around said first pin (P1), said second pin (P2), a third pin (P3) and a fourth pin (P4), the third pin (P3) being arranged between the first pin (P1) and the second pin (P2) along said periphery and the fourth pin (P4) being arranged between the first pin (P1) and the second pin (P2) and opposite the third pin (P3) along said periphery.
Item 62: The method according to item 61, wherein said guiding rope (300) is further moved around a fifth pin (P5), wherein the fifth pin (P5) guides said guiding rope (300) and/or said suture element (200) towards the fixing element (31), wherein the fifth pin (P5) is arranged adjacent to said first pin (P1).
Item 63: The method according to item 62, wherein said guiding rope (300) is further moved around a sixth pin (P6), wherein said guiding rope (300) and/or the suture element (200) is guided towards the fixing element (31) between the fifth pin (P5) and the sixth pin (P6).
Item 64: A method for assembling a medical implant (6) according to one of the items 17 to 21, comprising the steps of
   a. providing a suture element (200) comprising a connecting section (240),
   b. providing a medical textile (500), particularly comprising or consisting of a felt material,
   c. connecting the connecting section (240) of the suture element (200) to the medical textile (500),
   d. providing a suspension plate (100),
   e. engaging the suture element (200) with the suspension plate (100), particularly by the method according to one of the items 42 to 63.
Item 65: The method according to item 64, wherein the connecting section (240) of the suture element (200) is connected to the medical textile (500) by stitching.
Item 66: The method according to item 64, wherein the suture element (200) comprises or consists of a plurality of fibers (203), wherein the fibers (203) are separated from each other in the connecting section (240), and wherein the separated fibers (203) of the connecting section (240) are advanced into and/or through the medical textile (500) to connect the suture element (200) of the implantable suspension device (1) to the medical textile (300).
Item 67: The method according to item 66, wherein the connecting section (240) of the suture element (200) is connected to the medical textile (500) by repeatedly advancing a needle comprising at least one barb through the connecting section (240) and the medical textile (500) to advance the separated fibers (203) of the connecting section into and/or through the medical textile (500).
Item 68: The method according to one of the items 64 to 67, wherein, after connecting the connecting section (240) of the suture element (200) to the medical textile (500), the medical textile (500) and/or the suture element (200) is connected to an elongated flexible implant (G), particularly outside of a human or animal body.
Item 69: The method according to item 68, wherein the medical textile (500) and/or the suture element (200) is stitched to the implant (G).
Item 70: The method according to item 68, wherein the medical textile (500) is connected to the implant (G) by repeatedly advancing a needle comprising at least one barb through the medical textile (500) and the implant (G) and/or wherein the suture element (200) is connected to the implant (G) by repeatedly advancing a needle comprising at least one barb through the connecting section (240) of the suture element (200) and the implant (G).
Item 71: The method according to one of the items 68 to 70, wherein the medical textile (500) is at least partially wrapped around the implant (G) prior to connecting the medical textile (500) to the implant (G), particularly wherein the medical textile (500) is wrapped around the implant (G), such that the medical textile (500) assumes a tubular shape, wherein said implant (G) is connected to an inner surface of the medical textile (500).

### List of reference numerals

| | |
|---|---|
| 1 | Implantable suspension device |
| 2 | Assembly device |
| 3 | Screw |
| 3a | Tip |
| 3b | Cutting thread |
| 3c | Lateral opening |
| 4 | Anchor |
| 4a | Tooth |
| 4b | Groove |
| 4c | Lateral opening |
| 5 | Tool |
| 6 | Medical implant |
| 11 | First through-hole |
| 12 | Second through-hole |
| 13 | Third through-hole |
| 14 | Fourth through-hole |
| 15 | Fifth through-hole |
| 16 | Sixth through-hole |
| 21 | Holding part |
| 22 | Casing part |
| 22a | First hole |
| 22b | Second hole |
| 22c | wall |
| 22d | opening |
| 23 | Guide bar |
| 23a | Slot |
| 23b | hole |
| 23c | Notch |
| 23d | Tip |
| 24 | Handle part |
| 24a | Handle |
| 24b | Groove |
| 25 | Tip part |
| 26 | Inner thread |
| 27 | Outer thread |
| 28 | Actuating element |
| 30 | First part |
| 31 | Fixing element |
| 32 | Second handle |
| 33 | Further handle |
| 34 | Brace |
| 41 | First locking loop |
| 42 | Second locking loop |
| 50 | Second part |
| 60 | Lid part |
| 61 | First handle |
| 62 | First hole |
| 63 | Second hole |
| 64, 65, 66, 67 | Further hole |
| 68 | Further handle |
| 100 | Suspension plate |
| 100a | Front side |
| 100b | Rear side |
| 200 | Suture element |
| 201 | First end section |
| 201a | First end |
| 202 | Second end section |
| 202a | Second end |
| 203 | Fiber |
| 204 | Fiber end |
| 231 | First suspension section |
| 232 | Second suspension section |
| 240 | Connecting section |
| 250 | Needle |
| 300 | Guide rope |
| 301 | First end of the guiding rope |
| 302 | Second end of the guiding rope |
| 303 | Loop |
| 305 | Inner volume |
| 306 | Funnel |
| 307 | Opening of the funnel |
| 400 | Holder |
| 500 | Medical textile |
| 600 | Bone |
| 601 | Bone tunnel |
| 602 | First segment |
| 603 | Second segment |
| P1 | First pin |
| P2 | Second pin |
| P3 | Third pin |
| P4 | Fourth pin |
| P5 | Fifth pin |
| P6 | Sixth pin |
| P7 | Seventh pin |
| d | diameter |
| L | Longitudinal axis |
| L1 | First loop |
| L2 | Second loop |
| R | Rotation axis |
| A1 | First axis |
| F_{L} | Locking force |
| G | Implant |
| S | Stitches |

## Claims

1. A medical implant (6) comprising an implantable suspension device (1) for fixing an elongated flexible implant (G) in a desired position, the implantable suspension device (1) comprising a suspension plate (100) and a suture element (200) engaging with the suspension plate (100), wherein the suture element (200) comprises a first end section (201) extending from said suspension plate (100) to a first end (201a) of said suture element (200) and a second end section (202) extending from said suspension plate (100) to a second end (202a) of said suture element (200), wherein the second end section (202) comprises a connecting section (240) configured to be connected to said implant (G) or to a medical textile (500), and wherein the medical implant further comprises a medical textile (500), particularly comprising or consisting of a felt material, wherein the connecting section (240) of the suture element (200) is connected to the medical textile (500), wherein the medical textile (500) is configured to be connected to said implant (G).

2. The medical implant according to claim 1 **characterized in that** said connecting section (240) is positioned at the second end (202a) of the suture element (200).

3. The medical implant according to claim 1 or 2, **characterized in that** the suture element (200) comprises a needle (250) attached to the suture element (200), particularly to the first end (201a) or the second end (202a).

4. The medical implant according to one of the preceding claims, **characterized in that** the suture element (200) forms a first locking loop (41) for pressing the first end section (201) of the suture element (200) against the suspension plate (100) and thereby locking the first end section (201) with respect to the suspension plate (100).

5. The medical implant according to one of the preceding claims, **characterized in that** the second end section (202) comprises an adjustable first suspension section (231) between said suspension plate (100) and said connecting section (240), wherein the first suspension section (231) is configured to be shortened by pulling on the first end section (201) of the suture element (200), wherein particularly said first end section (201) protrudes out of said first locking loop (41).

6. The medical implant according to one of the preceding claims, **characterized in that** the suspension plate (100) is connected to or integrally formed with a screw (3) or an anchor (4) configured to be inserted into a bone.

7. The medical implant (6) according to one of the preceding claims, **characterized in that** the connecting section (240) is connected to the medical textile (500) by stitches (S).

8. The medical implant (6) according to one of the preceding claims, **characterized in that** the medical textile (500) comprises a tubular shape, wherein the medical textile (500) comprises an inner surface configured to be connected to said implant (G).

9. A method for assembling a medical implant (6) according to one of the claims 17 to 21, comprising the steps of
a. providing a suture element (200) comprising a connecting section (240),
b. providing a medical textile (500), particularly comprising or consisting of a felt material,
c. connecting the connecting section (240) of the suture element (200) to the medical textile (500),
d. providing a suspension plate (100),
e. engaging the suture element (200) with the suspension plate (100).

10. The method according to claim 9, wherein the connecting section (240) of the suture element (200) is connected to the medical textile (500) by stitching.

11. The method according to claim 9, wherein the suture element (200) comprises or consists of a plurality of fibers (203), wherein the fibers (203) are separated from each other in the connecting section (240), and wherein the separated fibers (203) of the connecting section (240) are advanced into and/or through the medical textile (500) to connect the suture element (200) of the implantable suspension device (1) to the medical textile (300).

12. The method according to claim 11, wherein the connecting section (240) of the suture element (200) is connected to the medical textile (500) by repeatedly advancing a needle comprising at least one barb through the connecting section (240) and the medical textile (500) to advance the separated fibers (203) of the connecting section into and/or through the medical textile (500).

13. The method according to one of the claims 9 to 12, wherein, after connecting the connecting section (240) of the suture element (200) to the medical textile (500), the medical textile (500) and/or the suture element (200) is connected to an elongated flexible implant (G), particularly outside of a human or animal body.

14. The method according to claim 13, wherein the medical textile (500) and/or the suture element (200) is stitched to the implant (G).

15. The method according to one of the claims 13 to 14, wherein the medical textile (500) is at least partially wrapped around the implant (G) prior to connecting the medical textile (500) to the implant (G), particularly wherein the medical textile (500) is wrapped around the implant (G), such that the medical textile (500) assumes a tubular shape, wherein said implant (G) is connected to an inner surface of the medical textile (500).
